# EUROPEAN PATENT APPLICATION

(11) **EP 3 009 077 A1**
(43) Date of publication of application: **20.04.2016**
(21) Application number: 14835141.4
(22) Date of filing: 05.06.2014
(51) Int. Cl.: A61B 17/00, A61B 1/00

(54) **PNEUMOPERITONEUM APPARATUS**

(30) Priority: 06.08.2013 JP 2013163551
(71) Applicant: OLYMPUS CORPORATION, Shibuya-ku Tokyo 151-0072 (JP)
(72) Inventor: HIRAGA, Kunitoshi, Tokyo 151-0072 (JP); FURUKAWA, Nobuyuki, Tokyo 151-0072 (JP)
(74) Representative: Schicker, Silvia
(86) International application number: PCT/JP2014/064938
(87) International publication number: WO 2015/019695

(57) **Abstract**

An insufflation apparatus includes a gas-feeding-conduit connecting section for connecting a conduit that feeds a predetermined gas to a subject, a first conduit that connects a gas-feeding-source connecting section and the gas-feeding-conduit connecting section, a suction-conduit connecting section for connecting a conduit that sucks the predetermined gas from an inside of the subject, a pump for circulating the predetermined gas to the subject, a conduit for circulation that connects the suction-conduit connecting section and the pump, a second conduit that connects a suction-source connecting section and the conduit for circulation, and a control section that controls a switching unit to operate in a first operation mode for circulating the predetermined gas to the subject via the pump and a second operation mode for sucking the predetermined gas to the suction source side via the second conduit.

## Description

### Technical Field

The present invention relates to an insufflation apparatus that performs insufflation in a body cavity.

### Background Art

In recent years, a surgical operation under an observation by a laparoscope has been widely adopted. In such a surgical operation by the laparoscope, an insufflation apparatus is used in order to feed a predetermined gas such as carbon dioxide into an abdominal cavity and secure an observation visual field by the laparoscope and a region in performing an operation.

When treatment is performed using an electric knife, for a case in which smoke or the like occurs in the abdominal cavity and prevents an observation, the smoke occurring in the abdominal cavity is sucked by a suction apparatus and exhausted to an outside of the abdominal cavity. However, since pressure of the gas in the abdominal cavity drops because of the suction, it is necessary to feed new carbon dioxide from a cylinder functioning as a gas feeding source. There is a disadvantage in that a consumed amount of the carbon dioxide increases.

Japanese Translation of PCT application No. 2013-505812 serving as a conventional example for solving such the disadvantage of the increase in the consumed amount of the carbon dioxide discloses a configuration for, without releasing the sucked carbon dioxide to an atmosphere, circulating the gas with a fluid pump in an apparatus including a filter and returning the gas, from which the smoke is removed by the filter, into the abdominal cavity.

The conventional example is a configuration for circulating the sucked carbon dioxide serving as the predetermined gas and reusing the carbon dioxide. Therefore, the conventional example can reduce the consumed amount of the carbon dioxide. However, the conventional example cannot cope with a case in which a large amount of smoke occurs in the abdominal cavity. Therefore, there is a demand for an insufflation apparatus that can further cope with even the case in which a large amount of smoke occurs in the abdominal cavity.

The present invention has been devised in view of the point explained above and it is an object of the present invention to provide an insufflation apparatus that can be used in an operation mode in which a predetermined gas is circulated and reused and can be also used in an operation mode corresponding to a case in which a large amount of smoke occurs in an abdominal cavity.

### Disclosure of Invention

### Means for Solving the Problem

An insufflation apparatus according to an aspect of the present invention includes: a gas-feeding-source connecting section connected to a gas feeding source that feeds a predetermined gas; a gas-feeding-conduit connecting section for connecting a gas feeding conduit that feeds the predetermined gas to a subject; a first conduit that connects the gas-feeding-source connecting section and the gas-feeding-conduit connecting section; a suction-conduit connecting section for connecting a suction conduit that sucks the predetermined gas from an inside of the subject; a pump for circulating the predetermined gas to the subject; a conduit for circulation that connects the suction-conduit connecting section and the pump; a connection conduit that connects the pump and the first conduit; a suction-source connecting section connected to a suction source for sucking the predetermined gas; a second conduit that connects the suction-source connecting section and the conduit for circulation; a switching unit that selectively switches a state in which the predetermined gas sucked from the suction-conduit connecting section is fed to the second conduit and a state in which the predetermined gas is fed to the first conduit via the pump; and a control section that controls the switching unit to operate in a first operation mode for circulating the predetermined gas to the subject via the pump and a second operation mode for sucking the predetermined gas to the suction source side via the second conduit.

### Brief Description of the Drawings

Fig. 1 is a diagram showing an overall configuration of an endoscope operation system including an insufflation apparatus in a first embodiment of the present invention;
Fig. 2A is a diagram showing an overall configuration of the insufflation apparatus in the first embodiment of the present invention;
Fig. 2B is a diagram showing an overall configuration of an insufflation apparatus in a first modification of the first embodiment of the present invention;
Fig. 3 is a diagram showing a structure near a suction cap receiver, a structure of a filter, and the like in the case of the first modification;
Fig. 4 is a diagram showing, in a table format, various operation modes and operation states of devices configuring the insufflation apparatus such as a suction apparatus and a flow rate adjustment valve related to the operation modes;
Fig. 5 is a flowchart showing processing content of a representative operation in the first embodiment;
Fig. 6 is a diagram showing a configuration of an insufflation apparatus in a second modification of first embodiment of the present invention;
Fig. 7 is a diagram showing a configuration of an insufflation apparatus in a third modification of the first embodiment of the present invention;
Fig. 8 is a diagram showing, in a table format, operation states of devices related to respective operation modes in the case of the third modifiation;
Fig. 9 is a diagram showing a configuration of an insufflation apparatus in a fourth modification of the first embodiment of the present invention;
Fig. 10 is a diagram showing, in a table format, operation states of devices related to respective operation modes in the case of the fourth modification;
Fig. 11 is a diagram showing an overall configuration of an insufflation apparatus in a second embodiment of the present invention;
Fig. 12 is a diagram showing a structure near a suction cap receiver and a structure of a filter in the second embodiment;
Fig. 13 is a diagram showing, in a table format, operation states of devices related to respective operation modes in the second embodiment;
Fig. 14A is a flowchart showing processing of monitoring/determination of a filtering characteristic of a filter;
Fig. 14B is a diagram showing a configuration of a characteristic determination circuit that determines the filtering characteristic of the filter;
Fig. 14C is a diagram showing a configuration for performing monitoring/determination of the filtering characteristic of the filter using a flow rate sensor;
Fig. 15 is a flowchart showing processing content for performing detection of mounting (connection) of the filter using the flow rate sensor;
Fig. 16 is an explanatory diagram of a configuration for performing detection of mounting (connection) of the filter different from the detection in Fig. 15;
Fig. 17 is an explanatory diagram of a configuration of a connector receiver in an apparatus main body;
Fig. 18 is an explanatory diagram of a configuration of a gas feeding & suction tube apparatus detachably connected to the connector receiver shown in Fig. 17;
Fig. 19 is a diagram showing a sectional structure of a gas feeding & suction tube;
Fig. 20 is an explanatory diagram of a configuration of a gas feeding & suction tube apparatus having a structure different from a structure shown in Fig. 18;
Fig. 21 is a diagram showing a tube connector having a shape different from a shape shown in Fig. 18;
Fig. 22 is an explanatory diagram of a configuration in which a filter in the tube connector shown in Fig. 18 is disposed on an outside; and
Fig. 23 is a diagram showing an overall configuration of an insufflation apparatus in a modification of the second embodiment of the present invention.

### Best Mode for Carrying Out the Invention

Embodiments of the present invention are explained below with reference to the drawings.

### (First Embodiment)

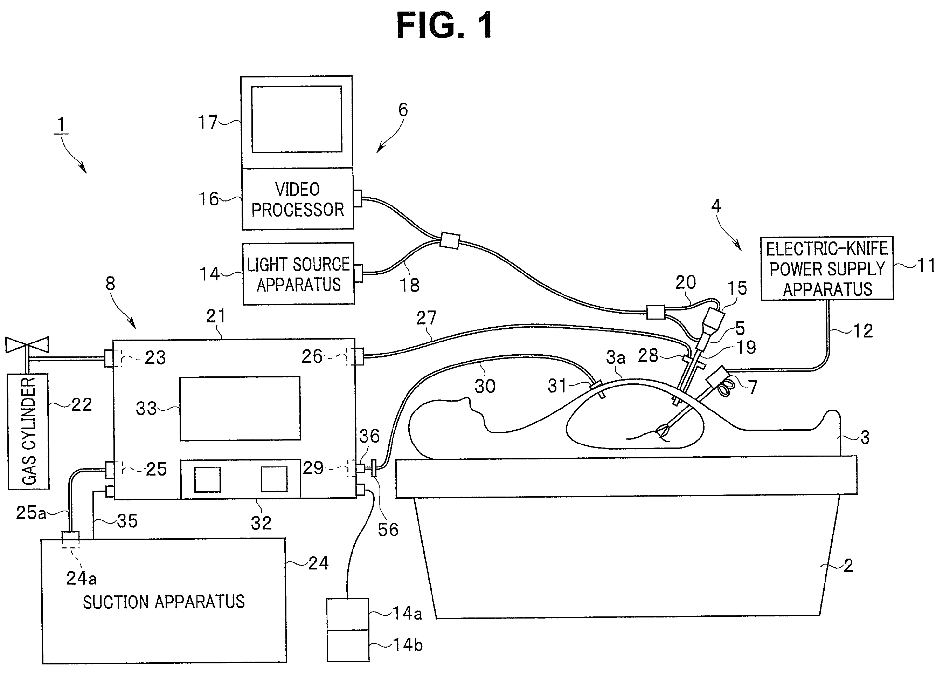

As shown in Fig. 1, an endoscope operation system 1 includes an electric knife apparatus 4 functioning as a surgical apparatus for performing an operation on a patient 3 treated as a subject placed on a bed 2, an endoscope apparatus 6 for observing a surgical operation using an endoscope 5, and an insufflation apparatus 8 in a first embodiment that performs insufflation in an abdomen 3 a set as an operation target in order to secure a visual field by the endoscope 5 and a region to be operated by an electric knife 7. Note that the abdomen 3a may be defined to form the subject.

The electric knife apparatus 4 is configured by an electric-knife power supply apparatus 11 that generates high-frequency power and the electric knife 7 to which the high-frequency power generated by the electric-knife power supply apparatus 11 is supplied via a cable 12. A surgeon can feed a high-frequency current to a diseased part grasped by, for example, a bipolar electrode at a distal end of the electric knife 7 and perform treatment such as cautery by operating a switch provided in a grasping section or the like of the electric knife 7. The electric knife 7 is inserted into the abdomen 3a by a not-shown trocar.

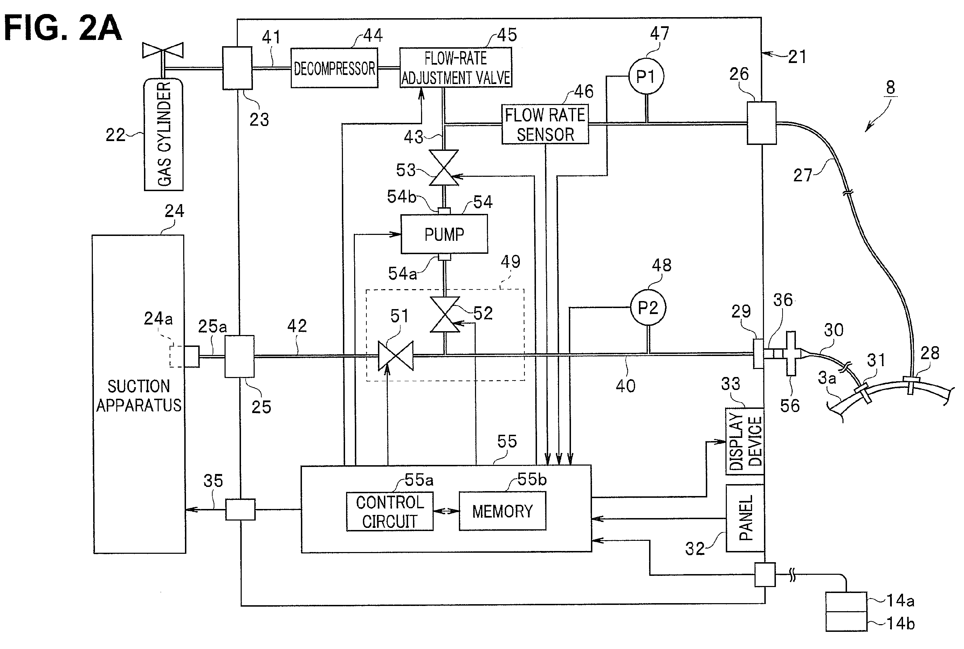

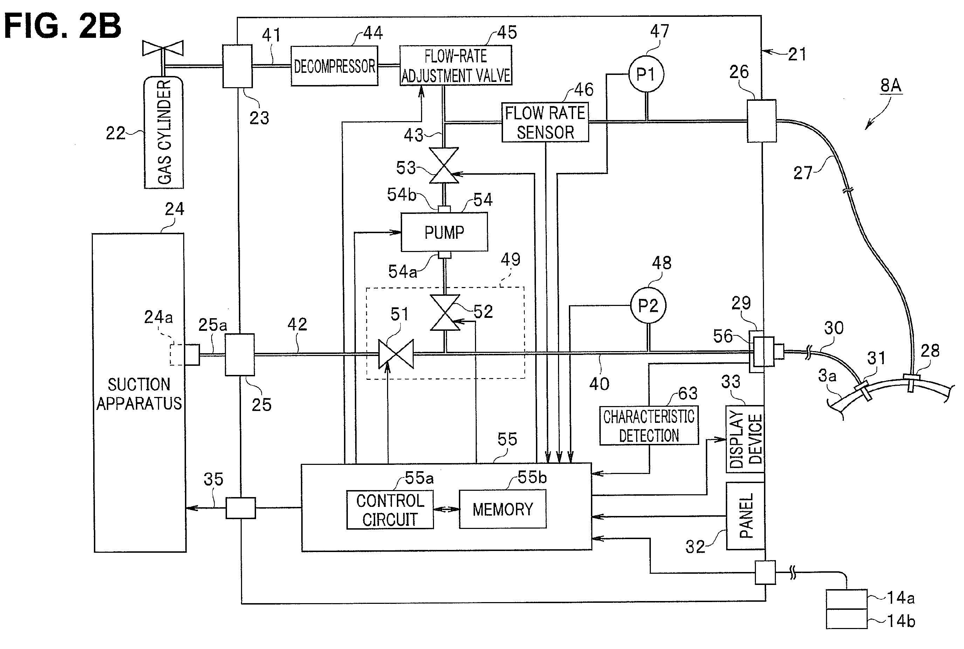

The endoscope apparatus 6 includes the endoscope 5 (on which an endoscope camera 15 is mounted) inserted into the abdomen 3a via a first trocar 28, a light source apparatus 34 that supplies illumination light to the endoscope 5, a video processor 16 functioning as a signal processing apparatus that performs signal processing on the endoscope camera 15 in which an image pickup apparatus mounted on an eyepiece section of the endoscope 5 is housed, and a monitor 17 functioning as an endoscopic-image display apparatus that displays, according to an input of a video signal generated by the video processor 16, an endoscopic image picked up by the image pickup apparatus in the endoscope camera 15. Note that, in Fig. 2 and subsequent figures, only the trocar 28 shown in Fig. 1 is schematically shown.

The light source apparatus 34 supplies (transmits) the illumination light generated by the light source apparatus to the endoscope 5 via a light guide cable 18. The illumination light supplied via the light guide cable 18 is transmitted via a not-shown light guide in an insertion section 19 of the endoscope 5 and emitted to the outside from a not-shown illumination window at a distal end of the insertion section 19 to illuminate a diseased part or the like in the abdomen 3a.

An object such as the illuminated diseased part is formed as an optical image by an objective lens provided in an observation window. The optical image is transmitted to the eyepiece section, picked up by the image pickup apparatus in the endoscope camera 15, and photoelectrically converted. An image pickup signal picked up by the image pickup apparatus is inputted to the video processor 16 via an image pickup cable 20. After signal processing on the image pickup signal is performed by the video processor 16, an endoscopic image is displayed on the monitor 17. Note that an endoscope incorporating an image pickup device at a distal end portion of the insertion section 19 may be used instead of the endoscope 5 on which the endoscope camera 15 is mounted.

An insufflation apparatus main body (abbreviated as apparatus main body) 21 covered with a housing configuring the insufflation apparatus 8 includes a connection cap receiver 23 functioning as a gas-feeding-source connecting section for connection to a carbon dioxide cylinder (abbreviated simply as gas cylinder or cylinder) 22, which stores, in a high pressure state, carbon dioxide, functioning as a gas feeding source (or supply source) that feeds (supplies) the carbon dioxide serving as a predetermined gas for performing insufflation in the abdomen 3 a. The apparatus main body 21 is connected to the gas cylinder 22 via a connection tube connected to the connection cap receiver 23.

The apparatus main body 21 includes a connection cap receiver 25 functioning as a suction-source connecting section for connection to a suction apparatus 24 functioning as a suction source that sucks the carbon dioxide serving as the predetermined gas. The apparatus main body 21 is connected to a suction cap receiver 24a of the suction apparatus 24 via a connection tube 25a connected to the connection cap receiver 25. Note that the suction apparatus 24 is configured by, for example, including a fluid pump having a suction ability larger than a suction ability of a fluid pump 54 provided in the apparatus main body explained below.

After adjusting the carbon dioxide stored by the gas cylinder 22 to a proper flow rate suitable for gas feeding, the apparatus main body 21 can feed, from a gas-feeding cap receiver 26 functioning as a gas-feeding conduit connecting section, via a gas feeding tube 27 having flexibility configuring a gas feeding conduit, one end of which is connected to the gas-feeding cap receiver 26, the carbon dioxide to the first trocar 28 side functioning as a trocar for gas feeding to which the other end of the gas feeding tube 27 is connected.

The first trocar 28 is needled into the abdomen 3 a. A hollow section of the gas feeding tube 27 communicates with an inside of the abdomen 3a, that is, an inside of an abdominal cavity via the first trocar 28. Insufflation can be performed in the abdominal cavity of the patient 3 with the carbon dioxide by feeding the carbon dioxide to the first trocar 28 side via the gas feeding tube 27 connected to the gas-feeding cap receiver 26 in a gas feeding mode explained below.

In the apparatus main body 21, a suction cap receiver 29 functioning as a suction-conduit connecting section is provided to make it possible to reduce pressure in the abdominal cavity and suck and remove smoke that occurs when treatment is performed by the electric knife 7. The suction cap receiver 29 is connected to, via a suction tube 30 having flexibility configuring a suction conduit, a second trocar 31 functioning as a trocar for suction to which the other end of the suction tube 30 is connected.

The second trocar 31 is needled into the abdomen 3a. A hollow section of the suction tube 30 communicates with the inside of the abdomen 3 a, that is, the inside of the abdominal cavity via the second trocar 31. The second trocar 31 makes it possible to suck the carbon dioxide in the abdominal cavity to the apparatus main body 21 side via the suction tube 30 in a circulation mode or the like explained below.

On a front surface of the apparatus main body 21, an operation panel 32 (in the figures, abbreviated as panel) for performing various kinds of operation and setting and a display device 33 that performs display of pressure in the abdominal cavity, a gas feeding flow rate, and the like are provided. The apparatus main body 21 includes foot switches 14a and 14b for performing instruction operation for gas feeding, suction, and smoke exhaust according to foot stepping operation by a user such as a surgeon and a communication cable 35 for transmitting a signal for performing ON/OFF of a suction operation to the suction apparatus 24. Note that the insufflation apparatus 8 may be defined as an apparatus including, besides the apparatus main body 21, the gas feeding tube 27 and the suction tube 30 connected to the apparatus main body 21 or may be defined to include a gas feeding source and a suction source.

Fig. 2A shows a configuration of an inside of the apparatus main body 21 and shows an overall configuration of the insufflation apparatus 8. As shown in Fig. 2A, the connection cap receiver 23 and the gas-feeding cap receiver 26 provided on an outer surface of the housing are connected by a conduit for gas feeding 41 functioning as a first conduit provided on an inside of the housing. The suction cap receiver 29 and the connection cap receiver 25 provided on the outer surface of the housing are connected via a conduit for circulation 40 provided on the inside of the housing and a conduit for suction 42 functioning as a second conduit in which a switching unit 49 is provided at an intermediate portion. The conduit for gas feeding 41 and the conduit for circulation 40 are connected via the fluid pump 54, to which an end portion of the conduit for circulation 40 is connected, and a connection conduit 43 functioning as a third conduit.

In the conduit for gas feeding 41, a decompressor 44 that performs decompression, a flow-rate adjustment valve 45 that adjusts a flow rate (configuring a flow-rate changing section), a flow rate sensor 46 functioning as a sensor that detects a flow rate, and a first pressure sensor 47 that measures pressure are respectively disposed in order of closeness to the connection cap receiver 23.

A second pressure sensor 48 that measures pressure is disposed on the conduit for circulation 40. A first electromagnetic valve 51 configuring the switching unit 49 is disposed on the conduit for suction 42 configuring the second conduit.

In the conduit for circulation 40, a second electromagnetic valve 52 configuring the switching unit 49 is interposed at an intermediate portion. An end portion of the second electromagnetic valve 52 is connected to a suction port 54a of the fluid pump 54 (in the drawings such as Fig. 2A, abbreviated simply as pump) 54. A discharge port 54b of the fluid pump 54 is connected to one end of the connection conduit 43. A third electromagnetic valve 53 is disposed at an intermediate portion in the connection conduit 43. The fluid pump 54 incorporates a motor and the like, sucks fluid (in this case, gas such as carbon dioxide) from the suction port 54a, discharges the sucked fluid from the discharge port 54b, and feeds the fluid to the connection conduit 43 side.

Note that the other end of the connection conduit 43 merges in the conduit for gas feeding 41 between the flow-rate adjustment valve 45 and the flow rate sensor 46. The connection conduit 43 is also considered to connect the fluid pump 54 and the conduit for gas feeding 41 forming the first conduit.

The second pressure sensor 48 is provided on the conduit for circulation 40 in a position further on the suction cap receiver 29 side than the switching unit 49.

The first electromagnetic valve 51 is provided on the conduit for suction 42 further on the connection cap receiver 25 side than the conduit for circulation 40.

In the apparatus main body 21, a control board 55 for electrically controlling operations of the flow-rate adjustment valve 45, the first electromagnetic valve 51, the second electromagnetic valve 52, the third electromagnetic valve 53, and the fluid pump 54 is provided. The control board 55 includes a control circuit 55a that is configured by a central processing unit (abbreviated as CPU) and the like and controls the operations of the flow-rate adjustment valve 45 and the like and a memory 55b that stores program data for the CPU to perform a predetermined control operation and stores various control data and the like.

Detection value of a flow rate measured by the flow rate sensor 46, pressure values measured by the first pressure sensor 47 and the second pressure sensor 48, a signal inputted by operation of the operation panel 32, operation signals by the foot switches 14a and 14b, and the like are inputted to the control circuit 55a of the control board 55.

In the present embodiment, a suction cap 36, in which a filter 56 formed by fiber or the like having a fine mesh structure to perform filtering to prevent passage of particulates and the like other than carbon dioxide such as smoke mixed in the carbon dioxide sucked from the second trocar 31 side and allow clean carbon dioxide to pass is provided near and end portion of the suction tube 30, is detachably attached to the suction cap receiver 29. In the first embodiment in the present invention, the suction cap 36, in which the filter 56 is provided at an intermediate portion in the suction tube 30, is configured to be detachably connected to the suction cap receiver 29 of the apparatus main body 21.

As in a configuration of a first modification shown in Fig. 2B, the filter 56 may be configured to be detachably connectable to the suction cap receiver 29. An insufflation apparatus 8A in the first modification shown in Fig. 2B is different from the insufflation apparatus 8 in the first embodiment in that a filter mounting section is provided in the suction cap receiver 29 in the apparatus main body 21 in the first embodiment and a characteristic detection circuit 63 is provided in the apparatus main body 21.

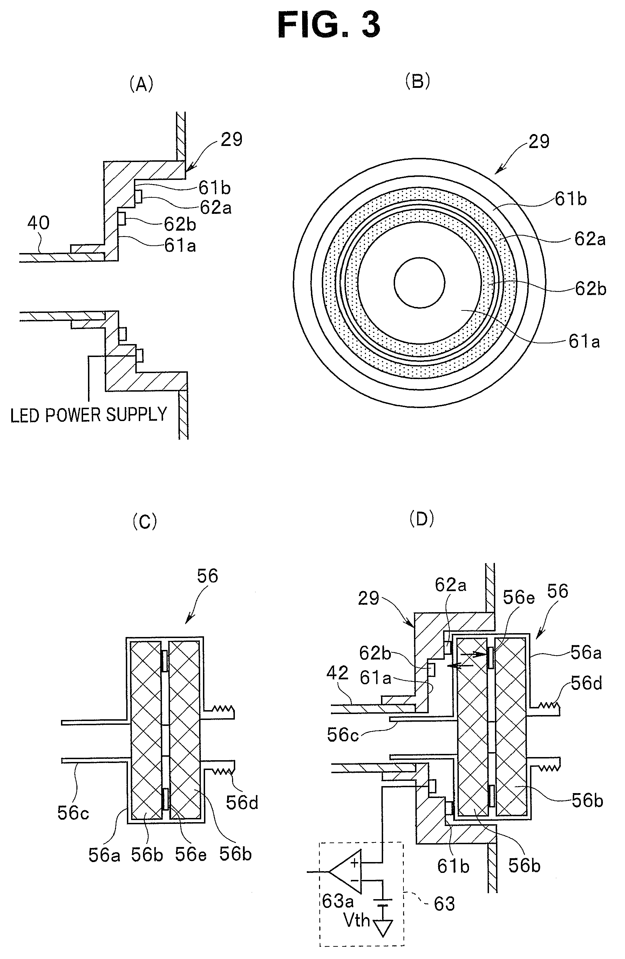

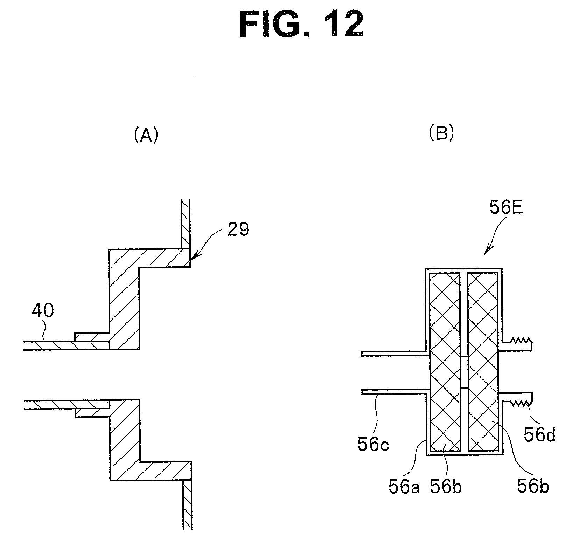

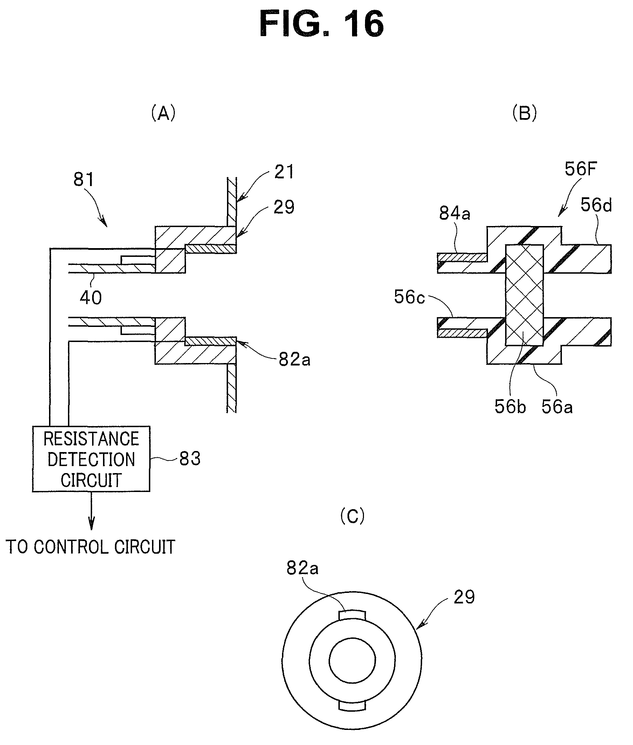

As explained above, in the case of the configuration of the first modification, the suction cap receiver 29 includes the filter mounting section on which the filter 56 is detachably mounted (connected). Note that the filter mounting section is not limited to the configuration shown in Fig. 3 and can also be configured as shown in Fig. 12 and Fig. 16 explained below. The filter 56 is a filter for filtering carbon dioxide sucked from the inside of the abdominal cavity into clean carbon dioxide. Therefore, a position where the filter 56 is disposed may be any position on the conduit for circulation 40 from an intermediate portion in the suction tube 30 or may be any position to the electromagnetic valve 53 or a check valve 74 explained below on the connection conduit 43 connected to the conduit for circulation 40. The filter 56 filters at least the predetermined gas flowing in the suction tube 30 functioning as the suction conduit from an inside of the patient 3 forming the subject.

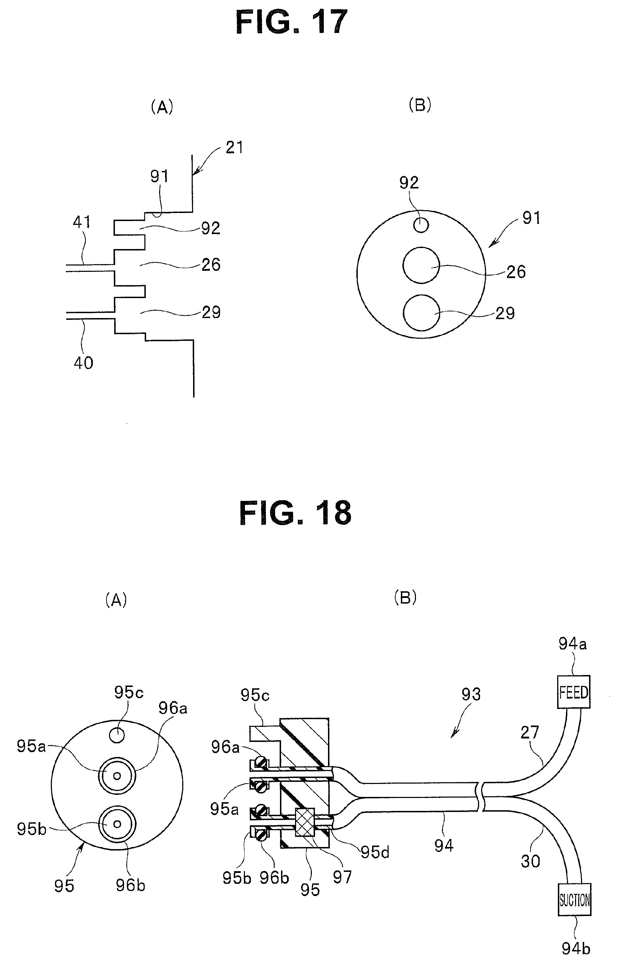

However, since the filter 56 needs to be replaced, when the filter 56 is provided on the apparatus main body 21 side, a position of the suction cap receiver 29, which is a place where the filter 56 is easily replaced, is a prospective position (place). Since the suction tube 30 is used to be disposed (disposable), the filter may be integrally provided on the suction tube 30 side as in the case of the first embodiment shown in Fig. 2. In this case, as a structure of the filter 56, a structure shown in Fig. 18 explained below may be adopted.

Note that, in Fig. 18, a configuration is shown in which the suction tube 30 is integrated with the gas feeding tube 27. However, a configuration may be adopted in which the suction tube 30 is separated from the gas feeding tube 27 and a filter 97 is provided at an intermediate portion in the suction tube 30.

Fig. 3 shows a structure near the suction cap receiver 29 in the first modification of the first embodiment. Note that Fig. 3(A) is a sectional view of the suction cap receiver 29 taken along a surface perpendicular to a center axis of the conduit for circulation 40. Fig. 3(B) is a side view of the suction cap receiver 29 viewed from a right side in Fig. 3(A). Fig. 3(C) is a sectional view of the filter 56. Fig. 3(D) is a sectional view of a state in which the filter 56 is mounted in a recess of the suction cap receiver 29. Note that the filter 56 is shown with hatching omitted.

As shown in Fig. 3(A), the suction cap receiver 29 includes a recess of a two-stage structure including a first step section 61 a of a ring shape at an end portion of the conduit for circulation 40 and a second step section 61b of a ring shape formed on an outer circumferential side of the first step section 61 a.

As shown in Fig. 3(B) as well, a light emitting diode (LED) 62a is disposed in the second step section 61b, for example, along the ring shape. A light receiving element 62b such as a solar cell is disposed in the first step section 61 a along the ring shape.

Note that the LED 62a is supplied with LED power source from the control board 55 to emit light. The LED 62a has a directional characteristic for emitting light in a direction substantially parallel to the center axis of the conduit for circulation 40. The light receiving element 62b also has a directional characteristic for receiving light from a direction substantially parallel to the center axis of the conduit for circulation 40 (an opposite direction of the emitting direction).

The filter 56 shown in Fig. 3(C) is detachably mounted on the suction cap receiver 29 including the recess of the two-stage structure.

In the filter 56 shown in Fig. 3(C), for example, two layers of filter disks 56b having a disk shape are disposed, via a spacer, in a filter case 56a fit in the second step section 61b as shown in Fig. 3(D). A cap for mounting 56c fit and mounted in the conduit for circulation 40 is provided in a center of one surface of the filter case 56a. A suction-tube attaching section 56d for connecting a connection cap provided at an end portion of the suction tube 30 is provided in a center of the other surface.

A mirror 56e of a ring shape is provided between the adjacent two filter disks 56b. The mirror 56e reflects light, which passes through the filter disks 56b from the LED 62a, to the light receiving element 62b side as shown in Fig. 3(D). Note that, when only a portion of the filter case 56a opposed to the LED 62a and the light receiving element 62b is transparent and the other portions are, for example, black, it is possible to reduce influence of external light.

The filter disks 56b have a characteristic of transmitting light of the LED 62a at a predetermined rate. When an amount of, for example, particulates such as smoke adhering to meshes configuring the filter disks 56b increases, a filtering characteristic is deteriorated and an output level of the light receiving element 62b decreases. In the present embodiment, focusing on a relation between the output level of the light receiving element 62b and the filtering characteristic of the filter 56, means for notifying replacement of the filter 56 when the filtering characteristic is deteriorated is provided as explained below.

A detection signal of the light receiving element 62b is inputted to the characteristic detection circuit 63. The characteristic detection circuit 63 includes a comparator 63a that compares an output signal level of the light receiving element 62b (in this case, an electromotive force corresponding to a received light amount) and a threshold Vth. When the output signal level is equal to larger than the threshold, the characteristic detection circuit 63 determines that the filter disks 56b are in a usable state in which the filter disks 56b do not need to be replaced. When the output signal level is smaller than the threshold, the characteristic detection circuit 63 outputs, to (the control circuit 55a of) the control board 55, a determination signal indicating that a filtering function of the filter disks 56b or the filter 56 is deteriorated.

When receiving the determination signal indicating that the filtering function is deteriorated, the control circuit 55a controls the display device 33 to display notification information for urging the user to replace the filter disks 56b or the filter 56. Therefore, the display device 33 forms a notifying section that performs notification for notifying the user such as the surgeon, who performs an operation, that the filtering function of the filter 56 is deteriorated and urging the user to replace the filter 56.

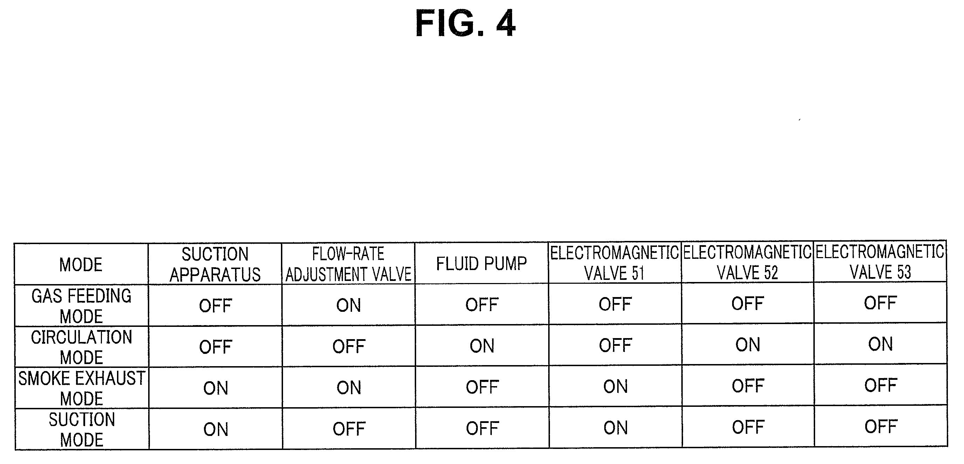

In the present embodiment and the first modification, the control circuit 55a functioning as the control means (or the control section) controls the suction apparatus 24, the flow-rate adjustment valve 45, the fluid pump 54, the first to third electromagnetic valves 51 to 53, and the like such that the insufflation apparatus 8 performs an operation of insufflation in four kinds of operation modes as shown in Fig. 4. Therefore, the control circuit 55a has a function of an operation-mode control section that controls a plurality of kinds of operation modes.

The four kinds of operation modes include a first operation mode (in other words, a circulation mode) for causing at least the fluid pump 54 to operate and feeding the carbon dioxide (serving as the predetermined gas) filtered by the filter 56 to be circulated to the first trocar 31 (or the subject) side via the gas feeding tube 27 (functioning as the gas feeding conduit) and a second operation mode (in other words a smoke exhaust mode) for stopping the operation of the fluid pump 54, sucking the carbon dioxide filtered by the filter 56 in the suction apparatus 24 functioning as the suction source, and exhausting the carbon dioxide.

When instruction operation for a gas feeding mode (serving as a third operation mode) is performed by the user, the control circuit 55a performs control to set only the flow-rate adjustment valve 45 in a predetermined operation state and turn off the suction apparatus 24, the fluid pump 54, and the first to third electromagnetic valves 51 to 53. Note that, when the control circuit 55a turns off the first to third electromagnetic valves 51 to 53, this means that the electromagnetic values are closed.

In the operation mode of the circulation mode, the control circuit 55a turns off the suction apparatus 24, the flow-rate adjustment valve 45, and the first electromagnetic valve 51 and turns on the fluid pump 54 and the second and third electromagnetic valves 52 and 53.

In the operation mode of the smoke exhaust mode, the control circuit 55a performs control in which ON and OFF in the circulation mode are interchanged. More specifically, the control circuit 55a turns on the suction apparatus 24, the flow rate adjustment valve 45, and the first electromagnetic valve 51 and turns off the fluid pump 54 and the second and third electromagnetic valves 52 and 53.

In the operation mode of a suction mode (serving as a fourth operation mode), the control circuit 55a performs control to turn off the flow-rate adjustment valve in the case of the smoke exhaust mode. More specifically, the control circuit 55a turns on the suction apparatus 24 and the first electromagnetic valve 51 and turns off the flow-rate adjustment valve 45, the fluid pump 54, and the second and third electromagnetic valves 52 and 53.

Note that, in the first modification shown in Fig. 2B, a configuration is shown in which the characteristic detection circuit 63 is provided on an outside of the control board 55. However, the characteristic detection circuit 63 may be provided on an inside of the control board 55.

In Fig. 1, Fig. 2A, and Fig. 2B, a configuration is shown in which the connection tube 25a connected to the connection cap receiver 25 of the apparatus main body 21 is connected to the suction apparatus 24 functioning as the suction source. However, a configuration may be adopted in which the connection tube 25a is connected to a suction apparatus provided on an outside of an operating room where an operation is performed.

The insufflation apparatus 8 in the present embodiment includes the connection cap receiver 23 functioning as a gas-feeding-source connecting section for connection to the gas cylinder 22 functioning as a gas feeding source that feeds, for example, carbon dioxide serving as a predetermined gas, the gas-feeding cap receiver 26 functioning as a gas-feeding-conduit connecting section for connecting the gas feeding tube 27 functioning as a gas feeding conduit that feeds the predetermined gas to (the abdomen 3a of) the patient 3 forming a subject, the conduit for gas feeding 41 functioning as a first conduit that connects the gas-feeding-source connecting section and the gas-feeding-conduit connecting section, the suction cap receiver 29 functioning as a suction-conduit connecting section for connecting the suction tube 30 functioning as a suction conduit that sucks the predetermined gas from the inside of the subject, the fluid pump 54 functioning as a pump for circulating the predetermined gas to the subject, the conduit for circulation 40 that connects the suction-conduit connecting section and the pump, the connection conduit 43 that connects the pump and the first conduit, the connection cap receiver 25 functioning as a suction-source connecting section connected to a suction source for sucking the predetermined gas, the conduit for suction 42 functioning as a second conduit that connects the suction-source connecting section and the conduit for circulation 40, the switching unit 49 that selectively switches a state in which the predetermined gas sucked from the suction-conduit connecting section is fed to the second conduit and a state in which the predetermined gas is fed to the first conduit via the pump, and the control board 55 or the control circuit 55a functioning as a control section that controls the switching unit 49 to operate in a first operation mode for circulating the predetermined gas to the subject via the pump and a second operation mode for sucking the predetermined gas to the suction source side via the second conduit.

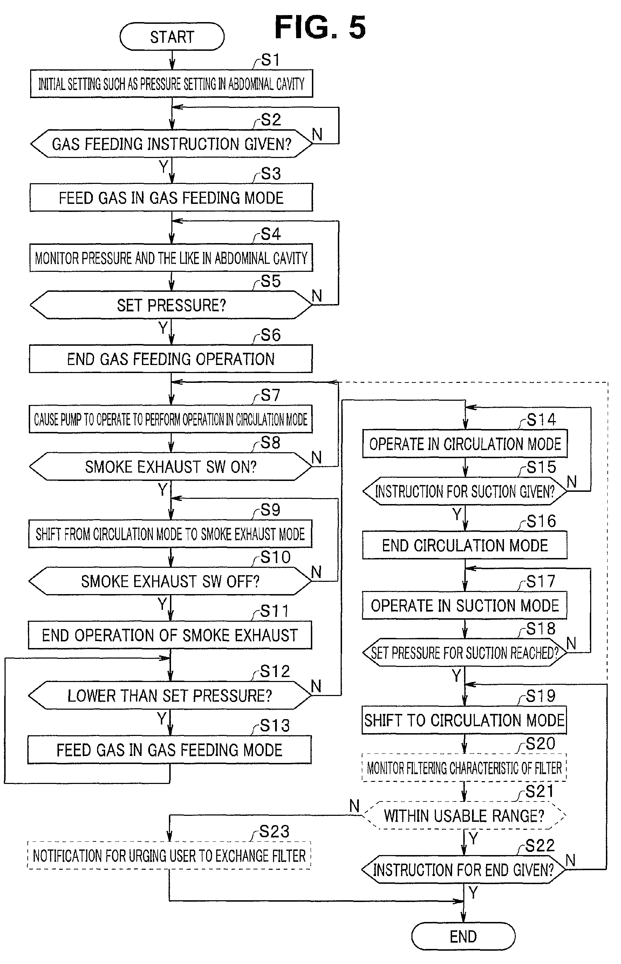

Representative operations in the present embodiment and the first modification are explained with reference to Fig. 5. After setting the insufflation apparatus 8 in the state shown in Fig. 1, the surgeon turns on a power supply of the insufflation apparatus 8. Then, each section of the insufflation apparatus 8 changes to an operable state. The control circuit 55a changes to a state in which the control circuit 55a reads program data stored in the memory 55b and controls operations of respective sections of the insufflation apparatus 8.

As shown in step S1 of Fig. 5, the surgeon performs, from the operation panel 32 or the like, initial setting such as pressure setting in an abdominal cavity and setting of a gas feeding flow rate in the case of gas feeding.

After the initial setting, as shown in step S2, the control circuit 55a changes to a state in which the control circuit 55a monitors a gas feeding instruction and waits for the gas feeding instruction. The surgeon performs operation of the gas feeding instruction from the operation panel 32 or depresses the foot switch 14a for gas feeding to perform the operation of the gas feeding instruction. Then, the control circuit 55a determines that the gas feeding instruction is given. As shown in step S3, the control circuit 55a performs control to perform gas feeding in the gas feeding mode corresponding to the gas feeding instruction.

More specifically, as shown in a table of Fig. 4, the control circuit 55a sets only the flow-rate adjustment valve 45 in the gas feeding mode to a predetermined operation state and performs control to turn off the suction apparatus 24, the fluid pump 54, and the first to third electromagnetic valves 51 to 53. In this case, after the carbon dioxide in the gas cylinder 22 is decompressed by the decompressor 44 and a passage flow rate is adjusted by the flow-rate adjustment valve 45, the carbon dioxide is fed via the gas feeding tube 27 and an inside of the abdominal cavity is subjected to insufflation by the fed carbon dioxide.

In this case, the flow rate sensor 46 measures a flow rate of the carbon dioxide flowing in the conduit for gas feeding 41 and sends a measured value to the control circuit 55a. The pressure sensor 48 measures pressure in the abdominal cavity via the suction tube 30 and sends a measured value to the control circuit 55a. As shown in step S4, the control circuit 55a monitors, for example, the pressure in the abdominal cavity according to the measurement values of the pressure sensor 48 and the like.

As shown in step S5, the control circuit 55a determines whether (the measurement value of) the pressure acquired by the pressure sensor 48 has reached the pressure set in the initial setting. When a determination result indicates that the pressure acquired by the pressure sensor 48 has not reached the set pressure, the control circuit 55a continues processing of the gas feeding by the gas feeding mode and monitors, for example, the pressure in the abdominal cavity.

On the other hand, when a determination result indicates that the pressure acquired by the pressure sensor 48 has reached the set pressure, as shown in step S5, the control circuit 55a ends the gas feeding operation. Consequently, it is possible to perform continuous gas feeding. It is possible to cause the inside of the abdominal cavity to quickly reach the set pressure (in a short time). On the other hand, conventionally, the pressure in the abdominal cavity is measured by the pressure sensor 47. Therefore, the gas feeding needs to be stopped and the gas is intermittently fed. Therefore, the conventional example has a disadvantage in that it takes time to cause the inside of the abdominal cavity to reach the set pressure.

After processing in step S5, as shown in step S6, the control circuit 55a causes the fluid pump 54 to operate and controls the fluid pump 54 to operate in the circulation mode. That is, the control circuit 55a changes the flow-rate adjustment valve 45 from ON to OFF and changes the second and third electromagnetic valves 52 and 53 from OFF to ON while keeping the suction apparatus 24 and the first electromagnetic valve 51 OFF as in the circulation mode shown in the table of Fig. 4.

In the case of the circulation mode, the carbon dioxide in the abdominal cavity is sucked by the fluid pump 54 via the suction tube 30. When the carbon dioxide is sucked, the carbon dioxide is filtered by the filter 56 into clean carbon dioxide. The clean carbon dioxide sucked by the fluid pump 54 is returned into the abdominal cavity via the conduit for gas feeding 41 and the gas feeding tube 27.

That is, the fluid pump 54 is set in the operation mode of the circulation mode. The clean carbon dioxide sucked from the inside of the abdominal cavity by the fluid pump 54 is returned into the abdominal cavity again. The carbon dioxide is circulated. Since the carbon dioxide in the abdominal cavity is filtered by filter 56 and circulated, it is possible to maintain a state of the carbon dioxide in the abdominal cavity in a cleaner state (than when the carbon dioxide is not circulated). A state in which the surgeon can easily perform an observation by the endoscope 5 is maintained.

In the circulation mode, as shown in step S8, the control circuit 55a determines whether an instruction switch for smoke exhaust is changed from OFF to ON. When the instruction switch for smoke exhaust is OFF, the control circuit 55a returns to processing in step S7 and continues the operation in the circulation mode.

In such a state, the surgeon can perform treatment on a diseased part using the electric knife 7 under an observation of the endoscope 5.

When the treatment is performed by the electric knife 7, since a high-frequency current is fed to a biological tissue of the diseased part or the like to cauterize the biological tissue, smoke occurs. In a state in which the occurring smoke is in a degree not preventing the observation of the endoscope 5, the surgeon continues the treatment in that state. However, when it is difficult to observe the diseased part or the like because of the occurring smoke, the surgeon presses, for example, the foot switch 14b with a foot as a switch for smoke exhaust and performs switch operation (ON) for a smoke exhaust instruction. According to the switch operation for smoke exhaust, the control circuit 55a determines that the instruction operation for smoke exhaust was performed, ends the circulation mode, and performs control to perform smoke exhaust in the smoke exhaust mode in step S9.

The control circuit 55a changes the fluid pump 54 and the second and third electromagnetic valves 52 and 53 from ON to OFF and changes the suction apparatus 24, the flow-rate adjustment valve 45, and the first electromagnetic valve 51 from OFF to ON as shown in the smoke exhaust mode in Fig. 4.

In the smoke exhaust mode, the carbon dioxide in the gas cylinder 22 is fed into the abdominal cavity via the gas feeding tube 27 in a state in which a flow rate is adjusted. The suction apparatus 24 is set in an operation state. The suction apparatus 24 sucks the carbon dioxide in the abdominal cavity via the suction tube 30, the conduit for circulation 40, the turned-on first electromagnetic valve 51, and the conduit for suction 42 forming the second conduit.

A suction function of the suction apparatus 24 is considerably larger than a suction function of the fluid pump 54 provided in the apparatus main body 21. Therefore, even if smoke occurs in the abdominal cavity, by sucking the carbon dioxide in the abdominal cavity, it is possible to quickly suck the smoke in the abdominal cavity simultaneously with the suction of the carbon dioxide in the abdominal cavity and discharge the smoke to the suction apparatus 24 side.

As shown in step S10, the control circuit 55a monitors whether the switch for smoke exhaust is turned off. In a state in which the switch for smoke exhaust is kept on, the control circuit 55a returns to the processing in step S9 and continues the operation in the smoke exhaust mode.

When the switch for smoke exhaust is turned off, the control circuit 55a ends the smoke exhaust mode as shown in step S 11. As shown in step S 12, the control circuit 55a determines whether, for example, pressure in the abdominal cavity measured by the pressure sensor 47 at time of an end of the smoke exhaust mode is lower than a set pressure.

In the case of a determination result indicating that the measured pressure in the abdominal cavity is lower than the set pressure, as shown in step S 13, the control circuit 55a sets the insufflation apparatus 8 in the gas feeding mode, feeds the carbon dioxide in the gas cylinder 22 into the abdominal cavity, and returns to processing in step S12. In this way, when, in the determination processing in step S12, obtaining a determination result indicating that the measured pressure in the abdominal cavity is not lower than the set pressure, as shown in step S14, the control circuit 55a sets the insufflation apparatus 8 in a state in which the insufflation apparatus 8 operates in the circulation mode. That is, the control circuit 55a causes the fluid pump 54 and the like to operate, sets the insufflation apparatus 8 in the setting state of the circulation mode explained above, and causes the insufflation apparatus 8 to operate in the circulation mode.

As shown in step S15, the control circuit 55a determines whether instruction operation for suction is performed. When the instruction operation for suction is not performed, the control circuit 55a returns to processing in step S14 and continues the operation in the circulation mode.

On the other hand, for example, when the instruction operation for suction by (the foot switch for suction in) the foot switch 14b is performed, as shown in step S16, the control circuit 55a ends the operation in the circulation mode. As shown in step S17, the control circuit 55a controls the insufflation apparatus 8 to operate in the suction mode. Note that, when the insufflation apparatus 8 is caused to operate in the suction mode, instruction setting for pressure to be set by suction from currently set pressure (hereinafter, set pressure for suction) is performed before this operation (e.g., during initial setting).

In the case of the suction mode, as shown in the suction mode in Fig. 4, the control circuit 55a turns on the suction apparatus 24 and the first electromagnetic valve 51, turns off the flow-rate adjustment valve 45, the fluid pump 54, and the second and third electromagnetic valves 52 and 53, and, in a state in which gas feeding of the carbon dioxide from the gas cylinder 22 is stopped, sucks the carbon dioxide in the body cavity with the suction apparatus 24.

When this suction operation is started, as shown in step S18, the control circuit 55a determines whether the pressure in the abdominal cavity measured by the pressure sensor 47 has reached the set pressure for suction. Therefore, when the pressure in the abdominal cavity measured by the pressure sensor 47 has not reached the set pressure for suction, the control circuit 55a returns to processing in the suction mode in step S17 and continues the operation of suction in the suction mode. On the other hand, when the measured pressure in the abdominal cavity has reached the set pressure for suction, the control circuit 55a proceeds to processing in step S19.

As shown in step S19, the control circuit 55a controls the insufflation apparatus 8 to shift from the suction mode to the circulation mode.

When the insufflation apparatus 8 shifts to the circulation mode, in the case of the configuration in the first embodiment, the control circuit 55a performs processing in step S22. On the other hand, in the case of the first modification, the control circuit 55a proceeds to processing in step S20 indicated by a dotted line.

As shown in step S20, the characteristic detection circuit 63 monitors the filtering characteristic of the filter 56. As shown in step S21 indicated by a dotted line, the characteristic detection circuit 63 determines whether the filtering characteristic is within a usable range (or within a range in which the filtering characteristic can be allowed) and sends a determination result to the control circuit 55a. In the case of a determination result indicating that the filtering characteristic is within the usable range in step S21, the control circuit 55a proceeds to processing in step S22.

In step S22, the control circuit 55a determines whether instruction operation for ending the insufflation apparatus 8 is performed by the surgeon. When the instruction operation for the end is not performed, the control circuit 55a continues the operation in the circulation mode of step S 19 or step S7 indicated by the dotted line. When the instruction operation for the end is performed, the control circuit 55a ends the processing shown in Fig. 5.

On the other hand, in the case of a determination result indicating the filtering characteristic of the filter 56 is deteriorated and the filtering characteristic is outside the usable range in the determination processing in step S21, in step S23 indicated by a dotted line, the control circuit 55a performs notification for urging the user to replace the filter 56 and ends the processing shown in Fig. 5. When the notification for urging the user to replace the filter 56 is performed, the surgeon replaces the filter 56 and performs same work from step S1 in Fig. 5 again. Note that, in a procedure shown in Fig. 5, the filtering characteristic of the filter 56 is monitored in step S20 in the case of the first modification and is determined in step S21. However, for example, the filtering characteristic may be monitored and further determined when the processing in step S1 is performed. The monitoring and the determination of the filtering characteristic may be performed anywhere between steps S1 to S22 in Fig. 5 other than step S1.

According to the present embodiment or the first modification in which the insufflation apparatus 8 operates as explained above, the insufflation apparatus 8 can be used in the circulation mode serving as the operation mode for circulating and reusing the carbon dioxide serving as the predetermined gas and can also be used in the smoke exhaust mode serving as the operation mode corresponding to a case in which a large amount of smoke occurs in the abdominal cavity. Therefore, even when treatment involving occurrence of smoke like treatment by the electric knife 7 is performed, it is possible to quickly eliminate the smoke and smoothly perform an operation under the observation of the endoscope 5. Even when the pressure in the abdominal cavity is changed to perform treatment, it is possible to cope with the treatment with simple operation.

According to the first modification of the present embodiment, the filtering characteristic of the filter 56 is monitored. When the filter 56 is clogged by smoke or the like and the filtering characteristic is deteriorated, the deterioration in the filtering characteristic can be notified to the user such as the surgeon. Therefore, it is possible to provide an environment in which the surgeon can smoothly perform an operation.

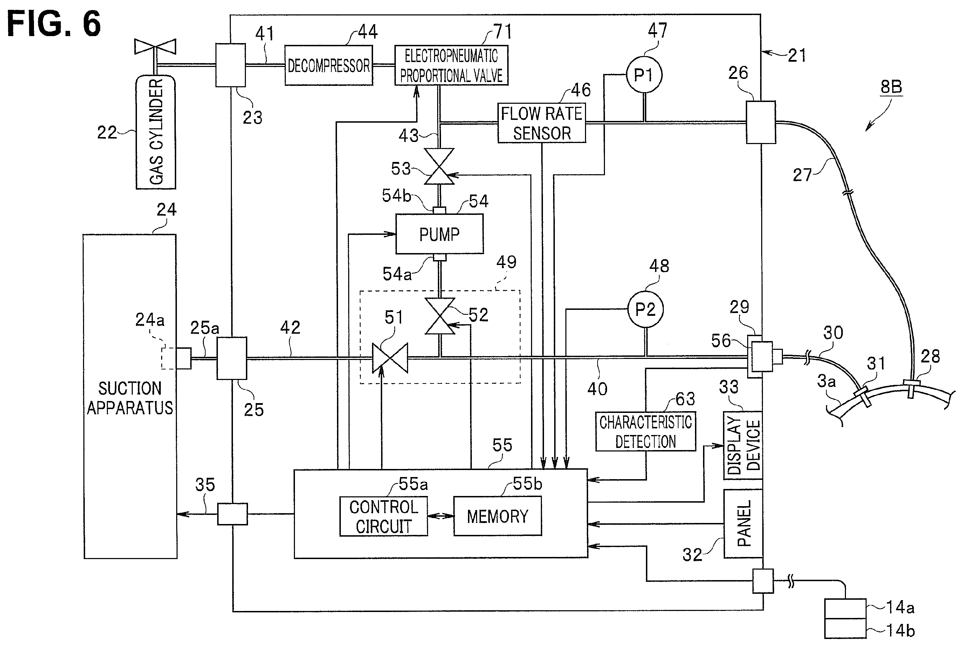

As a second modification in the present embodiment, a configuration shown in Fig. 6 may be adopted. An insufflation apparatus 8B shown in Fig. 6 has a configuration including the apparatus main body 21 in which the flow-rate adjustment valve 45 is replaced with an electropneumatic proportional valve 71 in the insufflation apparatus 8A having the configuration shown in Fig. 2B.

That is, in the first modification of the first embodiment, a flow rate is adjusted according to an electric signal by the flow-rate adjustment valve 45. However, in the second modification, pressure-adjusted carbon dioxide is outputted from the electropneumatic proportional valve 71 to the flow rate sensor 46 side by using the electropneumatic proportional valve 71. Therefore, this modification has action and effects substantially the same as action and effects of the first modification of the first embodiment because the flow rate (of the carbon dioxide) adjusted by the flow-rate adjustment valve 45 in the first modification of the first embodiment is replaced with pressure (of the carbon dioxide) adjusted by the electropneumatic proportional valve 71. Note that this modification is explained in a case in which this modification is applied to the insufflation apparatus 8A having the configuration shown in Fig. 2B. However, this modification can also be applied to the insufflation apparatus 8 having the configuration shown in Fig. 2A. In this case, this modification has action and effects substantially the same as action and effects of the first embodiment.

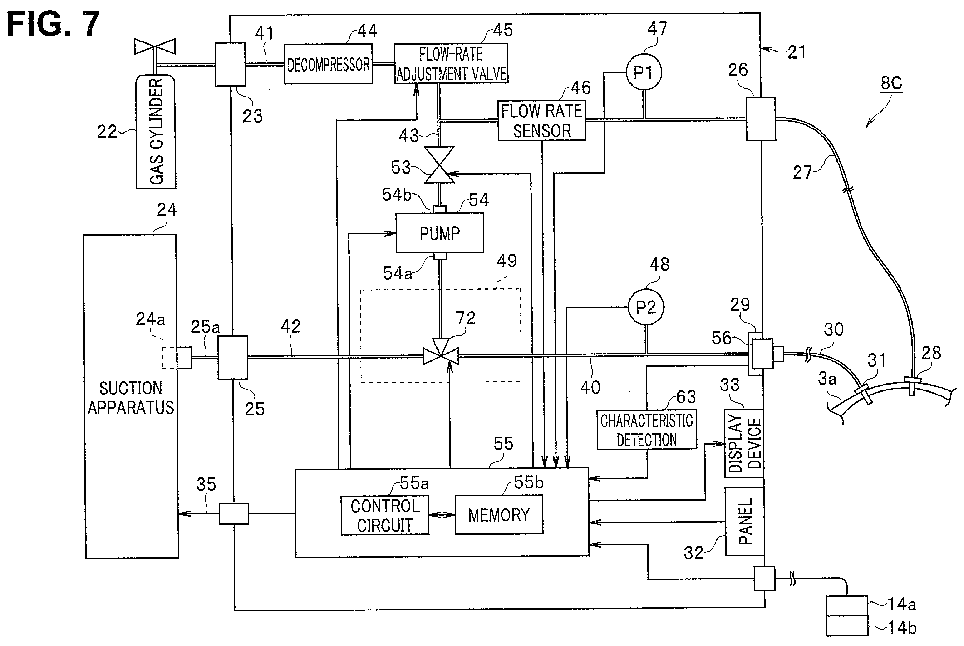

Fig. 7 shows an insufflation apparatus 8C in a third modification of the first embodiment. The insufflation apparatus 8C in the third modification shown in Fig. 7 includes the apparatus main body 21 in which the first electromagnetic valve 51 and the second electromagnetic valve 52 configuring the switching unit 49 is replaced with a three-way valve 72 in the insufflation apparatus 8A shown in Fig. 2B.

The three-way valve 72 has a function capable of selectively switching the conduit for circulation 40 on the suction cap receiver 29 side to at least a state in which the conduit for circulation 40 is allowed to communicate with the conduit for suction 42 on the connection cap receiver 25 side (a first communication state) and a state in which the conduit for circulation 40 is allowed to communicate with the fluid pump 54 (a second communication state). Note that the function for switching the conduit for suction 42 on the connection cap receiver 25 side to a state in which the conduit for suction 42 is allowed to communicate with the fluid pump 54 (a third communication state) is not always necessary. Therefore, a switching valve for selectively switching the first communication state and the second communication state may be used instead of the three-way valve 72. The switching valve or the three-way valve 72 is controlled to switch at least the first communication state and the second communication state by the control circuit 55a.

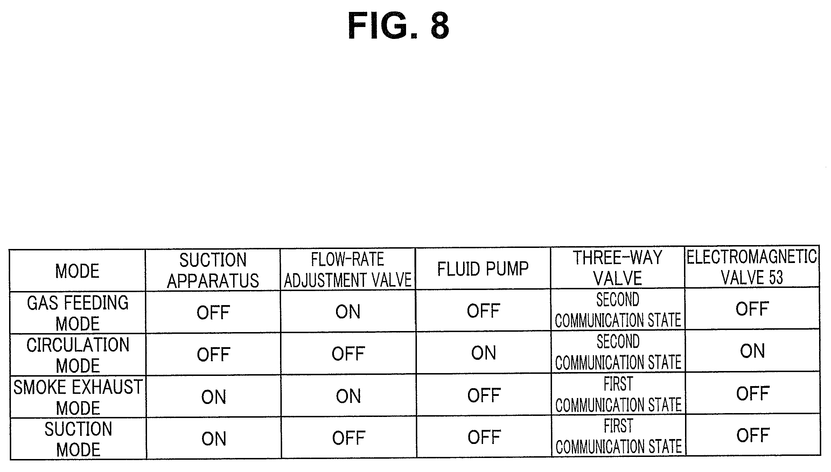

The three-way valve 72, the suction apparatus 24, and the like in the respective operation modes in the case of this modification are controlled as shown in Fig. 8 by the control circuit 55a. In Fig. 8, the first electromagnetic valve 51 and the second electromagnetic valve 52 in Fig. 4 are replaced with the three-way valve 72. The three-way valve 72 is set in the second communication state in the gas feeding mode and the circulation mode and set in the first communication state in the smoke exhaust mode and the suction mode by the control circuit 55a. Otherwise, Fig. 8 is the same as the content of Fig. 4.

This modification has action and effects substantially the same as the action and effects of the first modification of the first embodiment. Note that this modification is explained in the case in which this modification is applied to the insufflation apparatus 8A having the configuration shown in Fig. 2B. However, this modification can also be applied to the insufflation apparatus 8 having the configuration shown in Fig. 2A. In this case, this modification has action and effects substantially the same as the action and effects of the first embodiment.

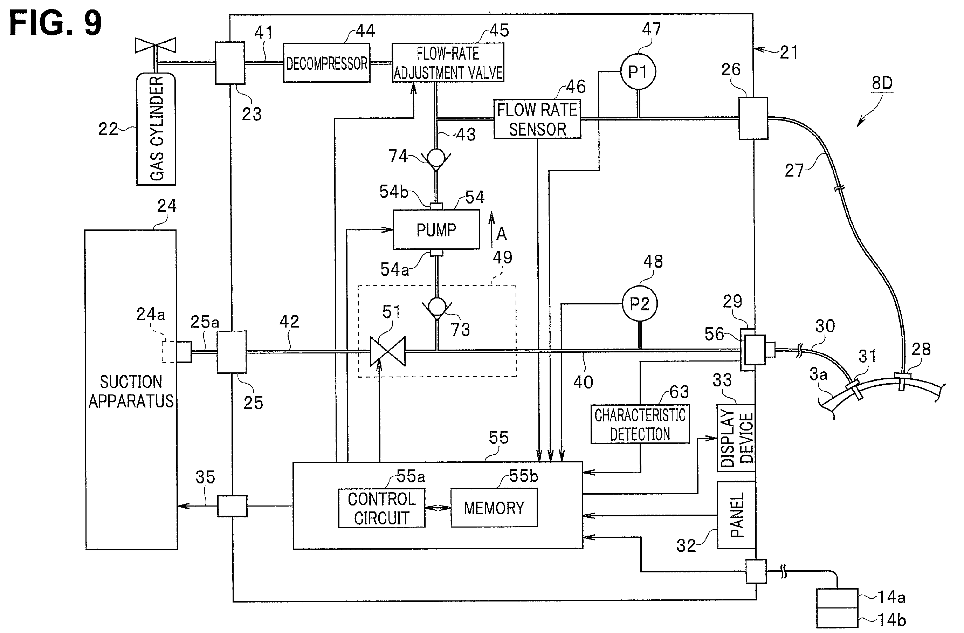

Fig. 9 shows an insufflation apparatus 8D in a fourth modification of the first embodiment. The insufflation apparatus 8D shown in Fig. 9 has a configuration including the apparatus main body 21 in which the second electromagnetic valve 52 and the third electromagnetic valve 53 are respectively replaced with check valves 73 and 74 in the insufflation apparatus 8A shown in Fig. 2B. The check valve 73 disposed on the conduit for circulation 40 and the check valve 74 disposed on the connection conduit 43 have a function of a check valve that allows gas flowing in one direction indicated by an arrow A on Fig. 9 to pass but inhibits passage of gas flowing in an opposite direction of the direction of the arrow A.

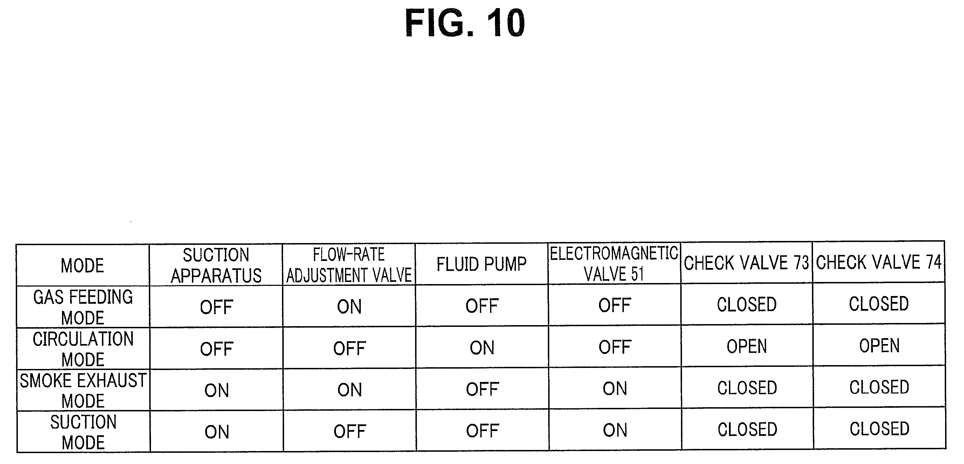

In the case of this modification, the control circuit 55a does not have to perform opening and closing of the second electromagnetic valve 52 and the third electromagnetic valve 53 in the first modification of the first embodiment. Control content of the control circuit 55a is more simplified. The check valves 73 and 74 in the respective operation modes in the case of this modification function as shown in Fig. 10.

In the case of the gas feeding mode, the check valves 73 and 74 respectively change to substantially closed operation state. Since the fluid pump 54 on a conduit for circulation 43 is turned off, the fluid pump 54 inhibits the carbon dioxide from passing through the conduit for circulation 40 and the connection conduit 43. Therefore, even if the check valves 73 and 74 are to open, since the fluid pump 54 inhibits the carbon dioxide from passing, when the fluid pump 54 is OFF, the check valves 73 and 74 respectively change to the substantially closed operation state.

On the other hand, in the case of the circulation mode, the fluid pump 54 is turned on and the check valves 73 and 74 are open to action of the carbon dioxide flowing in the direction of the arrow A and acts to circulate the carbon dioxide.

In the case of the smoke exhaust mode and the suction mode, as in the case of the gas feeding mode, since the fluid pump 54 is turned off, the check valves 73 and 74 respectively change to the substantially closed operation state. In the case of this modification, the control content by the control circuit 55a in the case of the first embodiment can be reduced. This modification can have action and effects substantially the same as the action and effects in the case of the first modification of the first embodiment. Note that this modification is explained in the case in which this modification is applied to the insufflation apparatus 8A having the configuration shown in Fig. 2B. However, this modification can also be applied to the insufflation apparatus 8 having the configuration shown in Fig. 2A. In this case, this modification has action and effects substantially the same as the action and effects of the first embodiment.

### (Second Embodiment)

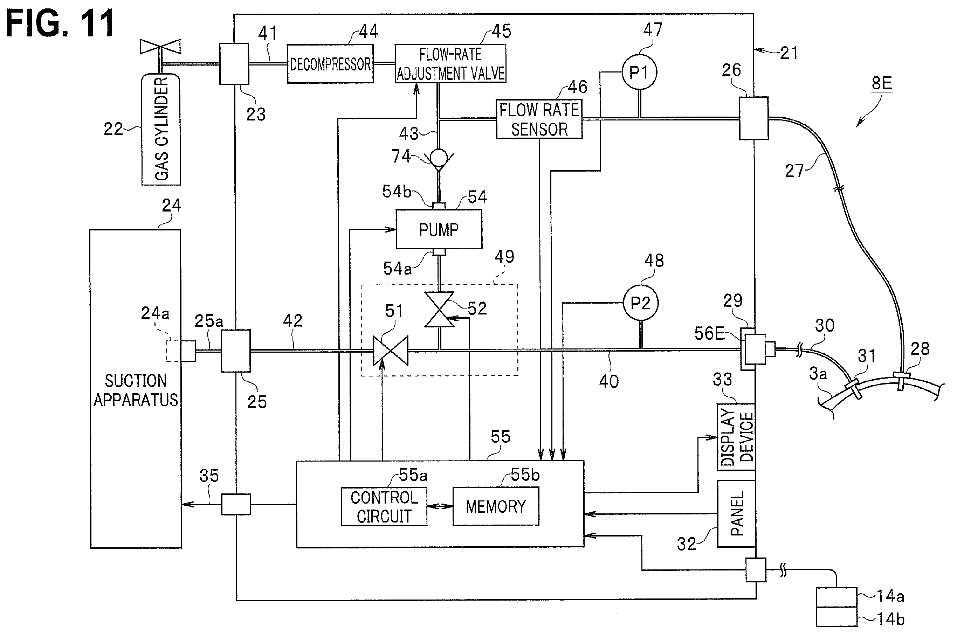

A second embodiment of the present invention is explained. Fig. 11 shows an insufflation apparatus 8E in a second embodiment of the present invention. The insufflation apparatus 8E includes the apparatus main body 21 in which the check valve 74 explained in Fig. 9 is used instead of the third electromagnetic valve 53 in the insufflation apparatus 8A shown in Fig. 2B. In the present embodiment, the insufflation apparatus 8E includes means for detecting deterioration in a filter characteristic of a filter 56E without using the characteristic detection circuit 63 shown in Fig. 2B.

Fig. 12 shows a structure near the suction cap receiver 29 in the present embodiment. Fig. 12(A) shows the structure near the suction cap receiver 29. Fig. 12(B) shows the filter 56E fit in the recess of the suction cap receiver 29 and detachably mounted.

In the first modification of the first embodiment, the suction cap receiver 29 is configured to include the recess of the two-stage structure. However, in the present embodiment, the suction cap receiver 29 is configured to include a recess of one stage. The LED 62a and the light receiving element 62b are not provided.

A filter 56E in the present embodiment has a structure not including the mirror 56e in the filter 56 shown in Fig. 3(C). In the present embodiment, the filter case 56a may be transparent or may be nontransparent.

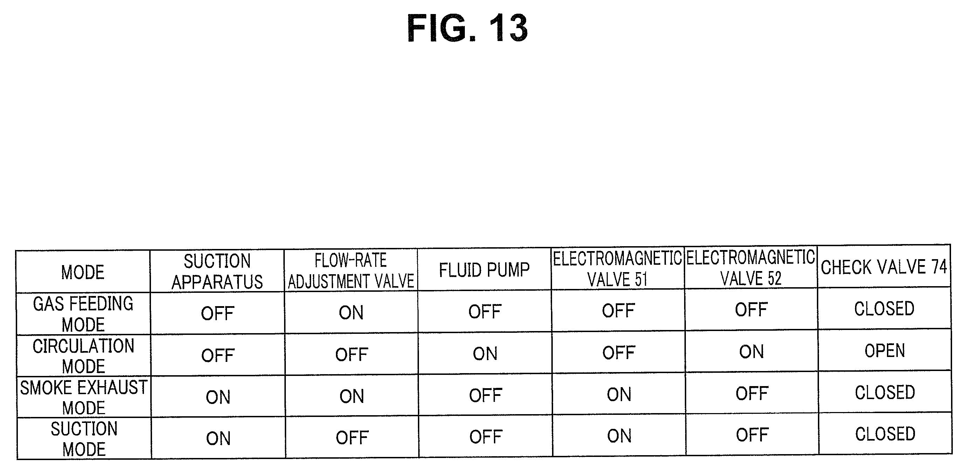

In the present embodiment, in the respective operation modes, the suction apparatus 24, the flow-rate adjustment valve, the fluid pump 54, the electromagnetic valves 51 and 52, and the check valve 74 change to open and close states shown in Fig. 13. In Fig. 13, contents are the same as the contents shown in Fig. 4 other than the check valve 74. The open and close states of the check valve 74 are the same as the open and close states shown in Fig. 10.

Representative operation content in the present embodiment is processing same as the processing shown in Fig. 5. However, in the present embodiment, control of the third electromagnetic valve 53 is not performed. The check valve 74 substitutes the function of the third electromagnetic valve 53.

More specifically, in the first embodiment, the control circuit 55a performed the control to open the third electromagnetic valve 53 only in the case of the circulation mode and close the third electromagnetic valve 53 in the other modes. On the other hand, in the present embodiment, the control circuit 55a performs control for changing the fluid pump 54 to an operation state (ON) only in the case of the circulation mode and turning off the fluid pump 54 in the other modes. In association with this control, the check valve 74 is open only in the case of the circulation mode and is substantially closed in the other modes.

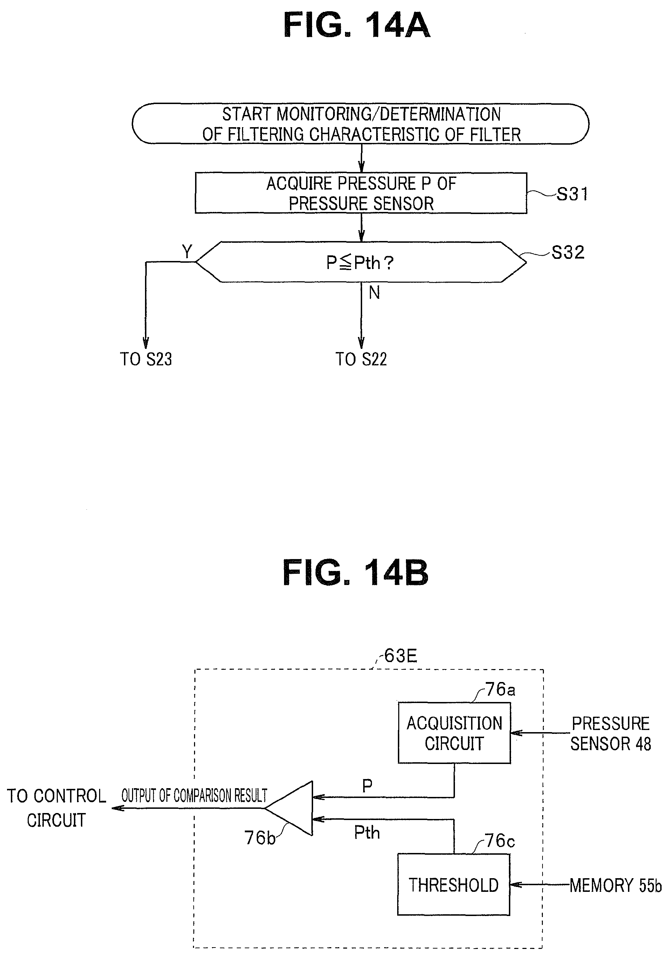

In the present embodiment, the processing for determining the filtering characteristic in steps S20 and S21 in Fig. 5 is performed. However, processing content for determination is different from the processing content in the first modification of the first embodiment. In the present embodiment, the filtering characteristic is determined in processing shown in Fig. 14A. In Fig. 14A, step S31 corresponds to the processing in step S20 in Fig. 5 and step S32 corresponds to the processing in step S21 in Fig. 5.

When the processing of monitoring and determination of the filtering characteristic is started, in step S31, the control circuit 55a captures a measurement value P of pressure measured by the pressure sensor 48 when the suction apparatus 24 is turned on. In step S32, the control circuit 55a compares the measurement value P with a threshold Pth stored in the memory 55b in advance. Note that, in this case, the flow-rate adjustment valve 45 is closed. The pressure sensor 47 measures pressure in the abdominal cavity.

In a state in which the pressure P indicates a certain value under a condition in which the suction ability of the suction apparatus 24 is set to a predetermined value using a new filter 56E or the filter 56E having the filtering characteristic without clogging, if clogging occurs in the filter 56E, a state equivalent to a state in which an opening amount of a conduit is reduced in a position where the filter 56E is disposed occurs. Therefore, the pressure P becomes smaller (than the certain value).

In other words, the pressure P of the pressure sensor 48 is reduced to pressure further reflecting suction pressure by the suction apparatus 24. Therefore, the threshold Pth serving as a boundary indicating that the filter 56E is desirably replaced is stored in the memory 55b in advance. When the pressure P is equal to or higher than the threshold Pth, the control circuit 55a determines that the filter 56E is within the usable range and shifts to the processing in step S22. Conversely, when the pressure P is smaller than the threshold Pth, the control circuit 55a shifts to the processing of notification for urging the user to replace the filter in step S23.

In the present embodiment, it is possible to determine the filtering characteristic of the filter 56E using the pressure P measured by the pressure sensor 48. Otherwise, the present embodiment has action and effects substantially the same as the action and effects of the first modification of the first embodiment.

Note that, instead of performing the processing shown in Fig. 14A, a characteristic determination circuit 63E shown in Fig. 14B may be provided. The characteristic determination circuit 63E shown in Fig. 14B includes an acquisition circuit 76a that acquires the pressure P of the pressure sensor 48 and a comparison circuit 76b that compares the pressure P with the threshold Pth. The comparison circuit 76b outputs a comparison result to the control circuit 55a. The control circuit 55a performs processing corresponding to a determination result by the characteristic determination circuit 63E. Note that the threshold Pth is stored in the memory 55b in advance as explained above. A threshold circuit (in the figure, abbreviated simply as threshold) 76c reads out the threshold Pth and outputs the threshold Pth to the comparison circuit 76b.

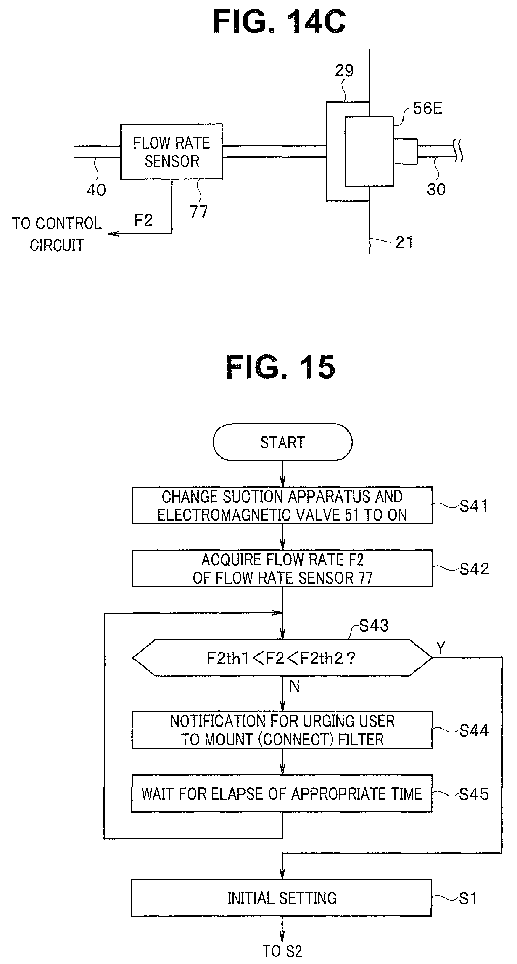

Note that a flow rate sensor 77 may be provided on the conduit for circulation 40 (instead of providing the pressure sensor 48) as explained in a modification shown in Fig. 14C. In this case, when the carbon dioxide is sucked by the suction apparatus 24, the control circuit 55a determines, according to whether a flow rate F2 measured by the flow rate sensor 77 is larger than a threshold Fth of a flow rate set in advance, whether the filter 56E is within the usable range.

When the filter 56E changes from a state in which the filter 56E is not clogged to a state in which the filter 56E is clogged, a flow rate of the carbon dioxide passing through the filter 56E decreases. Therefore, it is sufficient to set a threshold Fth of the flow rate in advance, determine a state in which the flow rate is smaller than a threshold Fth as a state of clogging, and urge the user to replace the filter 56E.

By using the flow rate sensor 77 shown in Fig. 14C, it is possible to configure means for detecting (determining) presence or absence of mounting (connection) concerning whether the filter 56E is mounted on the suction cap receiver 29. Note that, since outer diameters and the like of the cap for mounting 56c and the suction-tube attaching section 56d are different, unless in the filter 56E shown in Fig. 12 is interposed, the connection cap at the end portion of the suction tube 30 cannot be connected to the suction cap receiver 29. Therefore, in the case of the filter 56E of a type in which the filter disks 56b in the filter case 56a cannot be replaced, the means for detecting (determining) presence or absence of mounting (connection) concerning whether the filter 56E is mounted on the suction cap receiver 29 is unnecessary.

However, in a structure in which the filter disks 56b on the inside in the filter 56E can be replaced, it is likely that the filter 56E in a state in which the filter disks 56b are not inserted therein is interposed and the connection cap at the end portion of the suction tube 30 is connected to the suction cap receiver 29. In the case of the filter of the type in which the filter (the disks 56b) in the case can be replaced in this way, in some case, the connection cap at the end portion of the suction tube 30 is connected to the suction cap receiver 29 and used without using the filter.

In such a case, according to processing shown in Fig. 15 explained below, it is possible to detect whether the filter is substantially mounted (connected).

A power supply of an insufflation apparatus (8E) including the flow rate sensor 77 shown in Fig. 14C is turned on and the respective sections of the insufflation apparatus 8E change to an operation state. In the first step S41, the control circuit 55a sets the suction apparatus 24 and the first electromagnetic valve 51 to the operation state. Note that, when processing in step S41 is performed, an end of the suction tube 30 is changed to a state in which the end of the suction tube 30 is not connected to the second trocar 31 needled into the abdomen 3a of the patient 3 (e.g., a state in which the end of the suction tube 30 is opened to an atmosphere).

In the next step S42, the control circuit 55a acquires the flow rate F2 measured by the flow rate sensor 77.

In the next step S43, the control circuit 55a compares the acquired flow rate F2 and thresholds F2th1 and F2th2 of a flow rate set in advance and determines whether F2th1<F2<F2th2. Note that the threshold F2th2 is set to a value slightly larger than an upper limit value of a flow rate in a case in which a normal filter 56E is mounted (connected). The threshold F2th1 is set to a value slightly smaller than a lower limit value of the flow rate in the case in which the normal filter 56E is mounted (connected). Therefore, when the filter 56E in a state in which the filter disks 56b are inserted therein is mounted, a condition of F2th1<F2<F2th is satisfied.

When a determination result of F2th1<F2<F2th is not satisfied in determination processing in step S43, in the next step S44, the control circuit 55a performs notification for urging the user to mount (connect) the filter 56E. Thereafter, in the next step S45, control circuit 55a waits for elapse of appropriate time (e.g., 10 to 20 seconds) required for filter mounting (connection). After the elapse of the appropriate time in step S45, the control circuit 55a returns to the processing in step S43. On the other hand, in the case of a determination result satisfying F2th1<F2<F2th in the determination processing in step S43, the control circuit 55a determines that the filter 56E in which the filter disks 56b are inserted is mounted (connected) on the suction cap receiver 29. The surgeon connects the end of the suction tube 30 to the second trocar 31 and performs the processing of the initial setting in step S1 in Fig. 5.

By performing the processing of the mounting (connection) detection shown in Fig. 15 before an operation, there is an effect that it is possible to surely detect (determine) presence or absence of mounting (connection) of the filter 56E. It is possible to prevent contamination of the inside of the insufflation apparatus 8 in a case in which, for example, the insufflation apparatus 8 is used for an operation without mounting (connecting) the filter 56E.

Besides performing the detection of filter mounting (connection) with the configuration shown in Fig. 14C or the processing shown in Fig. 15, a configuration shown in Fig. 16 may be adopted to perform the detection of filter mounting (connection). However, in the configuration shown in Fig. 16, the filter disks 56b in the filter case 56a are an unreplaceable structure (a disposable type).

In the configuration shown in Fig. 16, an apparatus detecting apparatus 81 that detects whether the filter 56E is mounted on the suction cap receiver 29 of the apparatus main body 21 is provided. Fig. 16(A) shows a longitudinal section of the suction cap receiver 29. Fig. 16(B) shows, as a sectional view, a structure of a filter 56F detachably connected (mounted) to the suction cap receiver 29. Fig. 16(C) shows a front view of the suction cap receiver 29 viewed from a right side in Fig. 16(A).

As shown in Fig. 16(A), in the recess in the suction cap receiver 29, an inner circumferential surface having an inner diameter substantially equal to an outer diameter of the cap for mounting 56c of the filter 56F and fit and mounted in the cap for mounting 56c is formed. Mounting detecting sections 82a are provided in, for example, upper and lower positions in the inner circumferential surface using a conductor such as metal (see Fig. 16(C) as well).

The mounting detecting sections 82a are connected to, respectively via leads, a resistance detection circuit 83 that detects resistance (value).

On an outer circumference of the cap for mounting 56c in the filter 56F, resistors 84a formed in a ring shape and set to have a predetermined resistance value are provided. Therefore, when the cap for mounting 56c of the filter 56F is mounted in the recess of the suction cap receiver 29, the mounting detecting sections 82a in opposed positions conduct via the resistors 84a. The resistance detection circuit 83 changes to a state in which the resistance detection circuit 83 detects resistance of the resistors 84a. The resistance detection circuit 83 can surely detect according to the detected resistance value that the cap for mounting 56c of the filter 56F is mounted in the recess of the suction cap receiver 29.

Note that the filter 56F in the case of Fig. 16 has a structure in which one filter disk 56b is housed in the filter case 56a. However, the filter 56F may have the two-layer structure shown in Fig. 12 explained above. The suction-tube attaching section 56d is provided on a surface on an opposite side of the cap for mounting 56c in the filter case 56a. A hand-side end portion of the suction tube 30 is attached to the suction-tube attaching section 56d.

An outer diameter of the suction-tube attaching section 56d is set larger than the cap for mounting 56c. The suction-tube attaching section 56d is disabled to be mounted in the recess of the suction cap receiver 29 to configure a structure that can prevent wrong connection (wrong mounting) of the filter 56F to the suction cap receiver 29.

In the case of the configuration shown in Fig. 16, it is possible to surely detect whether the filter 56F is mounted. In the case of the configuration shown in Fig. 16, before an operation is actually performed, the control circuit 55a surely detects from the resistance value detected by the resistance detection circuit 83 whether the filter 56F is mounted. The configuration shown in Fig. 16 has a merit that it is unnecessary to cause the suction apparatus 24 and the like to operate as shown in Fig. 15 and to set the distal end of the suction tube 30 in the state in which the distal end is not connected to the second trocar 31.

Note that, as shown in Fig. 16, the outer diameter of the cap for mounting 56c in the filter 56F and the outer diameter or the like of the suction-tube attaching section 56d may be set to different sizes to configure a structure in which the suction tube 30 cannot be connected unless the filter 56F is mounted on the suction cap receiver 29.

In the first embodiment or the second embodiment (including the modifications) explained above, respective proximal ends of the gas feeding tube 27 and the suction tube 30 separate from each other are configured to be respectively detachably connected to the gas-feeding cap receiver 26 and the suction cap receiver 29 independently from each other. However, as explained below, the proximal ends of the gas feeding tube 27 and the suction tube 30 may be integrated to simplify connection work to configure a structure in which wrong connection is easily prevented.

Fig. 17(A) shows a schematic sectional shape of a tube connector receiver 91 to which a tube connector 95 of a gas feeding & suction tube apparatus 93 in the apparatus main body 21 is detachably connected. Fig. 17(B) shows a front view of the tube connector receiver 91 viewed from a right side thereof. Fig. 18(B) shows the entire gas feeding & suction tube apparatus 93 with a part thereof shown in a sectional view. Fig. 18(A) shows a front view of the tube connector 95 in the gas feeding & suction tube apparatus 93 shown in Fig. 18(B).

As shown in Fig. 17(A), a recess functioning as the tube connector receiver 91 is provided, for example, on a side surface of the apparatus main body 21. The gas-feeding cap receiver 26 and the suction cap receiver 29 are disposed adjacent to each other in an up-down direction on a bottom surface of the recess. A recess for protrusion reception 92 that prevents wrong connection in the case of connection and into which a protrusion 95c explained below is inserted is provided on an upper side of the gas-feeding cap receiver 26.

On the other hand, as shown in Fig. 18(B), the gas feeding & suction tube apparatus 93 includes a gas feeding & suction tube 94, in which the gas feeding tube 27 and the suction tube 30 are integrated, and a tube connector 95 integrally provided on a proximal end side of the gas feeding & suction tube 94 and respectively pressed into and mounted on the gas-feeding cap receiver 26 and the suction cap receiver 29. A distal end (end) side of the gas feeding & suction tube apparatus 93 is separated (divided) into the gas feeding tube 27 and the suction tube 30. A trocar connection cap for gas feeding 94a and a trocar connection cap for suction 94b are provided at separated respective distal ends of the gas feeding tube 27 and the suction tube 30.

As it is seen from Fig. 18(A) and Fig. 18(B), the tube connector 95 has a substantially disk shape and is set to an outer diameter fit in the recess of the tube connector receiver 91.

In the tube connector 95, a gas feeding cap 95a and a suction cap 95b respectively pressed into the gas-feeding cap receiver 26 and the suction cap receiver 29 of the tube connector receiver 91 are provided to project. Further, the protrusion 95c inserted into the recess for protrusion reception 92 is provided to project. Note that, when the protrusion 95c is inserted into the recess for protrusion reception 92, a lock mechanism that is not unlocked unless pulled out with force equal to or larger than a threshold may be provided.

The gas feeding cap 95a and the suction cap 95b are set to outer diameters respectively fit in inner diameters of the gas feeding cap 95a and the suction cap 95b. O-shaped rings 96a and 96b for hermetical sealing are housed in circumferential grooves provided on respective outer circumferential surfaces of the gas feeding cap 95a and the suction cap 95b.

The respective O-shaped rings 96a and 96b project to an outer side from the outer circumferential surfaces of the gas feeding cap 95a and the suction cap 95b. The gas feeding cap 95a and the suction cap 95b are respectively connected to the gas-feeding cap receiver 26 and the suction cap receiver 29 in a state in which the gas feeding cap 95a and the suction cap 95b are (the O-shaped rings 96a and 96b are respectively) compressed to keep airtightness when being respectively pressed into the gas-feeding cap receiver 26 and the suction cap receiver 29. When the gas feeding cap 95a and the suction cap 95b are connected, the protrusion 95c is inserted into the recess for protrusion reception 92. By adopting such a configuration, the gas feeding cap 95a and the suction cap 95b are enabled to be connected to the gas-feeding cap receiver 26 and the suction cap receiver 29 of the tube connector receiver 91 in one connection operation (work) without an error.

The filter 97 having a mesh shape is disposed at an intermediate portion in a tube (or a conduit) 95d, which communicates with the suction tube 30, on an inside of the tube connector 95 to enable carbon dioxide sucked from the distal end side of the suction tube 30 to be filtered into clean carbon dioxide.

Note that the trocar connection cap for gas feeding 94a is connected to the first trocar 28 functioning as the trocar for gas feeding. The trocar connection cap for suction 94b is connected to the second trocar 31 functioning as the trocar for suction. Marks "feed" and "suction" are added to the trocar connection cap for gas feeding 94a to make it possible to reduce an error in connection.

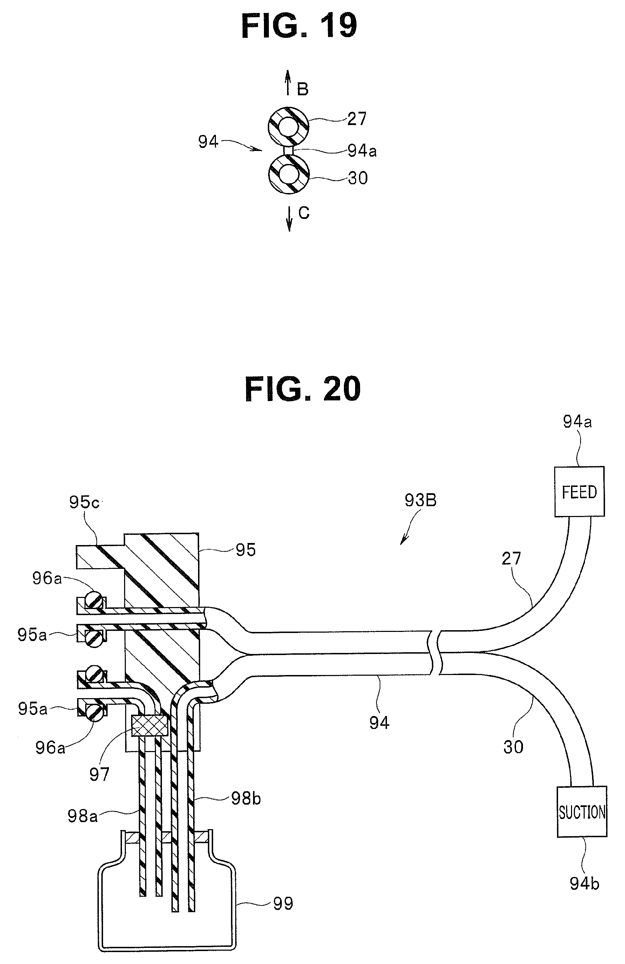

Fig. 19 shows a cross sectional structure of the gas feeding & suction tube 94.

As shown in Fig. 19, the gas feeding & suction tube 94 has a structure in which the gas feeding tube 27 and the suction tube 30 are connected (or integrated) adjacent to each other by a thin section 94c. Therefore, by applying force in directions in which both the tubes are separated (arrows B and C in Fig. 19), it is possible to break the connection by the thin section 94c and easily separate both the tubes. Therefore, when the gas feeding & suction tube 94 is actually used, the surgeon can separate the gas feeding tube 27 and the suction tube 30 in length for easy use and use the gas feeding & and suction tube 94.

With the configuration shown in Fig. 17 to Fig. 19, since the gas feeding tube 27 and the suction tube 30 are collected as one tube (excluding the end sides), the apparatus main body 21 of the insufflation apparatus can be easily connected to the gas-feeding cap receiver 26 and the suction cap receiver 29. In this case, the tube connector 95 can be connected to the tube connector receiver 91 in one place in the apparatus main body 21. Wrong connection can be surely prevented by the recess 92 and the protrusion 95c.

Since the marks are respectively added to the distal end sides of the gas feeding & suction tube 94, it is possible to prevent wrong connection in connecting the gas feeding & suction tube 94 to the trocars. It is possible to easily separate both the tubes. The surgeon can adjust length of separated portions to length for easy use and use the gas feeding & suction tube 94 according to preference of the surgeon, disposition in performing an operation, and the like.

As a modification of the gas feeding & suction tube apparatus 93 shown in Fig. 18, a configuration transformed as shown in Fig. 20 may be adopted.

In a gas feeding & suction tube apparatus 93B shown in Fig. 20, the tube 95d interposed with the filter 97 in the tube connector 95 in the gas feeding & suction tube apparatus 93 shown in Fig. 18 is changed to two trap bottle tubes 98a and 98b and projected from the tube connector 95. End portions of the projected trap bottle tubes 98a and 98b are inserted into a trap bottle 99. Note that, in this case, the filter 97 is disposed on the trap bottle tube 98a side communicating with the suction cap 95b. When body fluid, blood, and the like in the abdominal cavity are sucked by the suction apparatus 24 from the trocar connection cap for suction 94b side together with the carbon dioxide in the abdominal cavity, it is possible to store the sucked body fluid, blood, and the like in the trap bottle 99 and further guide the carbon dioxide serving as the predetermined gas to the trap bottle tube 98a side.

Otherwise, the configuration shown in Fig. 20 is the same as the configuration shown in Fig. 18. When the configuration shown in Fig. 20 is adopted, the trap bottle 99 is interposed at an intermediate portion in the suction tube 30. Therefore, it is possible not only to suck gas including smoke in the abdominal cavity via the suction tube 30 but also suck body fluid, blood, and the like. Note that the sucked body fluid, blood, and the like can be stored in the trap bottle 99. Further, smoke floating in the carbon dioxide can be removed by the filter 97. Otherwise, the configuration has effects same as the effects in the case of the configuration shown in Fig. 18.

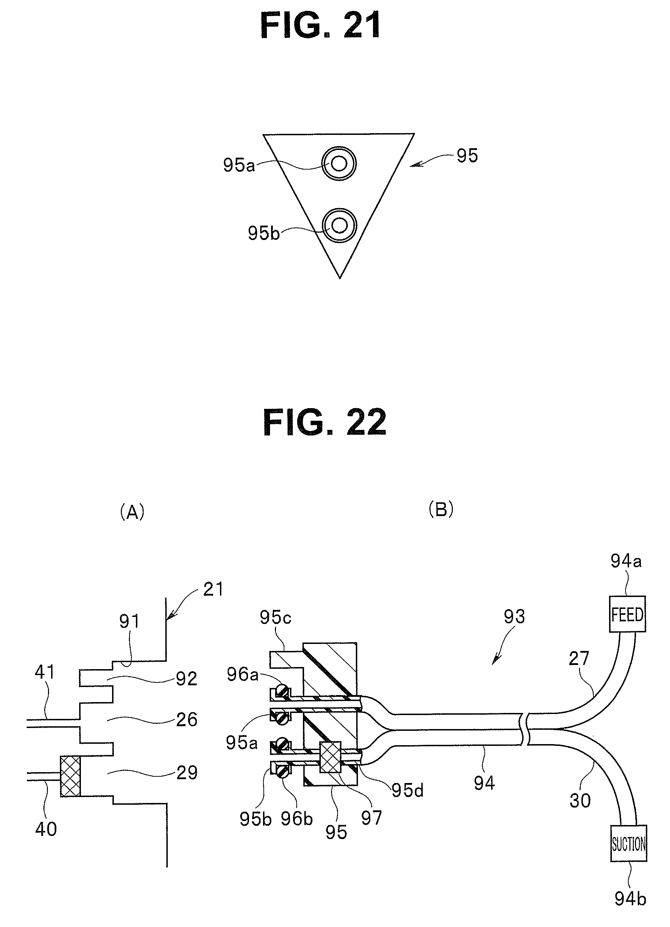

Note that, in the explanation with reference to Fig. 18 and the like, external shapes of the tube connector 95 and the tube connector receiver 91 are formed in circular shapes. However, as shown in Fig. 21, the tube connector 95 may be formed in, for example, a shape like a triangle. In this case, the protrusion 92 is unnecessary.

For example, a configuration may be adopted in which the filter 97 disposed in the tube connector 95 shown in Fig. 18 is not provided. Instead, a recess in which the filter 97 is mounted may be provided on the suction connector receiver 29 side shown in Fig. 17. Fig. 22 shows such a configuration.

In a tube connector receiver 91C shown in Fig. 22(A), in the tube connector receiver 91 shown in Fig. 18, depth of the recess of the suction connector receiver 29 is increased to provide a housing section in which the filter 97 is mounted. After the filter 97 is mounted in the housing section, the tube connector 95 shown in Fig. 22(B) of a structure in which the filter 97 is not inserted is mounted. The gas feeding & suction tube apparatus 93 shown in Fig. 22(B) has a configuration in which the filter 97 is not provided in the tube connector 18 in the gas feeding & suction tube apparatus 93 shown in Fig. 18. In such a case, the gas feeding & suction tube apparatus 93 has action and effects substantially the same as the action and effects in the case of the gas feeding & suction tube apparatus 93 shown in Fig. 18.

Note that, in the embodiments (including the modifications) explained above, the carbon dioxide serving as the predetermined gas is circulated using the fluid pump 54 provided in the apparatus main body 21 to make it possible to realize the apparatus main body 21 small in size and low in costs. However, as explained below, the suction apparatus 24 on the outside of the apparatus main body 21 may be used for the circulation of the carbon dioxide.

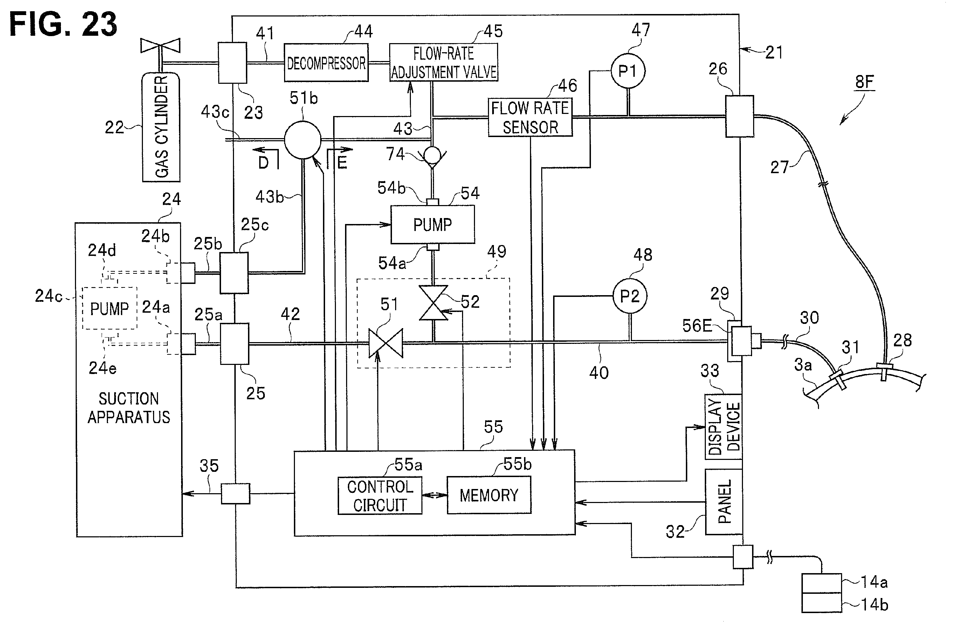

In an insufflation apparatus 8F shown in Fig. 23, for example, in the configuration of the insufflation apparatus 8E shown in Fig. 11, a discharge cap receiver (or an exhaust port receiver) 24b connected to a discharge port (or an exhaust port) 24d of a fluid pump 24c in the suction apparatus 24 is connected to a cap receiver 25c of the apparatus main body 21 via a connection tube 25b. In Fig. 23, the fluid pump (abbreviated simply as pump) 24c not shown in Fig. 11 is indicated by a dotted line. The cap receiver 25c is connected to, via a second conduit for circulation 43b, the conduit for gas feeding 41 or the conduit for circulation 43 in which the flow-rate adjustment valve 45 and the flow rate sensor 46 merge. A suction port 24e of the fluid pump 24c is connected to the suction cap receiver 24a. A switching valve 51b that performs switching of a conduit to communicate is provided at an intermediate portion in the second conduit for circulation 43b. The switching of the switching valve 51b is controlled by the control circuit 55a. Usually, the control circuit 55a sets the switching valve 51b in a state in which the conduit 43b on the cap receiver 25c side communicates with an exhaust conduit 43c side as indicated by an arrow D. In this state, when the fluid pump 24c of the suction apparatus 24 is set in the operation state, the sucked carbon dioxide is exhausted to the outside via the exhaust conduit 43c.

Only when the operation in the circulation mode is selected, the control circuit 55a switches the switching valve 51b such that the conduit for circulation 43b on the cap receiver 25c side communicates with the conduit for gas feeding 41 (or connection conduit 43) side as indicated by an arrow E. The control circuit 55a opens the first electromagnetic valve 51.

In this case, the carbon dioxide sucked by the fluid pump 24c of the suction apparatus 24 is fed to the conduit for gas feeding 41 (or connection conduit 43) side, caused to pass through the flow rate sensor 46, and fed to the gas feeding tube 27 side. That is, in this case, the carbon dioxide sucked by the suction apparatus 24 (the fluid pump on the inside) is circulated in parallel to the circulation of the carbon dioxide sucked by the fluid pump 54.

Otherwise, the configuration is the same as the configuration shown in Fig. 11. The configuration shown in Fig. 23 includes the suction apparatus 24 functioning as a suction source disposed on the outside of the housing of the apparatus main body 21, the second conduit for circulation 43b functioning as a fourth conduit, one end of which is connected to an exhaust port (discharge port) side for exhausting (discharging) a predetermined gas after suction in the suction source and the other end of which is connected to a first conduit in which a flow-rate changing section and the flow rate sensor 46 merge, the exhaust conduit 43c provided to branch on the fourth conduit and for exhausting the predetermined gas passed (or flowed) through the fourth conduit to the atmosphere, and the switching valve 51 b that selectively causes the predetermined gas passed through the fourth conduit to communicate with one of the first conduit side and the exhaust conduit 43c side. In the case of the circulation mode serving as a first operation mode, the control circuit 55a functioning as a control section performs control to circulate the predetermined gas sucked by the suction source to the first trocar 28 (or subject) side via the fourth conduit and the first conduit.

With the configuration shown in Fig. 23, the insufflation apparatus 8F is set in the circulation mode, besides circulating the carbon dioxide with the fluid pump 54 inside the apparatus main body 21, it is possible to circulate the carbon dioxide using the suction apparatus 24 (the fluid pump 24c on the inside) on the outside of the apparatus main body 21.

Therefore, a flow rate can be considerably increased to be larger than a flow rate of the carbon dioxide circulated in the circulation mode using only the fluid pump 54 inside the apparatus main body 21. Therefore, even when the electric knife 7 or the like is used and smoke occurs, it is possible to expand an application range in which measures can be taken to sufficiently reduce or eliminate the smoke through the circulation mode. Otherwise, the configuration has effects same as the effects in the case of Fig. 11.

Note that the insufflation apparatus 8F shown in Fig. 23 is explained as the configuration of the modification of the second embodiment. However, the configuration can also be applied to the insufflation apparatus 8 shown in Fig. 2 and other insufflation apparatuses.

In the embodiments including the modifications explained above, embodiments and the like configured by rearranging a part of the configurations of the embodiments also belong to the present invention. For example, the gas feeding tube 27 and suction tube 30 side in Fig. 23 may be formed as the gas feeding & suction tube apparatus 93 shown in Fig. 18. In this case, the apparatus main body 21 side is also formed in a structure to which the gas feeding & suction tube apparatus 93 can be connected. The gas feeding tube 27 and suction tube 30 side in Fig. 23 may be replaced with those shown in Fig. 20 and Fig. 22.

This application is based upon and claims the benefit of priority from Japanese Patent Application No. 2013-163551 filed in Japan on August 6, 2013, disclosed contents of which are incorporated in this specification, the claims, and the drawings.

## Claims

1. An insufflation apparatus comprising:
a gas-feeding-source connecting section for connecting to a gas feeding source that feeds a predetermined gas;
a gas-feeding-conduit connecting section for connecting a gas feeding conduit that feeds the predetermined gas to a subject;
a first conduit that connects the gas-feeding-source connecting section and the gas-feeding-conduit connecting section;
a suction-conduit connecting section for connecting a suction conduit that sucks the predetermined gas from an inside of the subject;
a pump for circulating the the predetermined gas to the subject;
a conduit for circulation that connects the suction-conduit connecting section and the pump;
a connection conduit that connects the pump and the first conduit;
a suction-source connecting section connected to a suction source for sucking the predetermined gas;
a second conduit that connects the suction-source connecting section and the conduit for circulation;
a switching unit that selectively switches a state in which the predetermined gas sucked from the suction-conduit connecting section is fed to the second conduit and a state in which the predetermined gas is fed to the first conduit via the pump; and
a control section that controls the switching unit to operate in a first operation mode for circulating the predetermined gas to the subject via the pump and a second operation mode for sucking the predetermined gas to the suction source side via the second conduit.

2. The insufflation apparatus according to claim 1, wherein the switching unit includes: an openable and closable first on-off valve provided on the second conduit on the suction-source connecting section side; and one of an openable and closable second on-off valve and a check valve that allows the predetermined gas to pass only in one direction from the conduit for circulation to the connection conduit side, the second on-off valve and the check valve being provided on the connection conduit.

3. The insufflation apparatus according to claim 1, wherein the switching unit is configured using a switching valve for selectively performing one of communication of the conduit for circulation, which communicates with the suction-conduit connecting section, with the second conduit on the suction-source connecting section side and communication of the conduit for circulation with the pump.

4. The insufflation apparatus according to claim 1, further comprising: a flow-rate changing section that is disposed on the first conduit and changes a flow rate of the predetermined gas; and a filter that is provided at an intermediate portion in the suction conduit or at an intermediate portion in the conduit for circulation and filters the predetermined gas into a clean predetermined gas, wherein
in a case of the first mode, the control section controls the flow-rate changing section to set a flow rate allowed to pass by the flow-rate changing section to 0, controls the switching unit to switch to a state in which the suction-conduit connecting section communicates with the pump, and performs control for setting the pump in an operation state to thereby circulate the predetermined gas filtered by the filter to the subject via the gas feeding conduit.

5. The insufflation apparatus according to claim 1, further comprising a flow-rate changing section that is disposed on the first conduit and changes a flow rate of the predetermined gas, wherein
in a case of the second mode, the control section controls the switching unit to switch to a state in which the suction-conduit connecting section communicates with the second conduit, sets the suction source in an operation state, sucks the predetermined gas in the subject to the suction source side through the second conduit, and performs control to set the predetermined gas by the gas feeding source to a flow rate adjusted by the flow-rate changing section and feed the predetermined gas into the subject via the gas feeding conduit.

6. The insufflation apparatus according to claim 1, further comprising a flow-rate changing section that is disposed on the first conduit and changes a flow rate of the predetermined gas, wherein
the control section stops the operation of the pump, stops the suction by the suction source, and controls the switching unit to operate in a third operation mode for setting the predetermined gas by the gas feeding source to a flow rate adjusted by the flow-rate changing section and feeding the predetermined gas into the subject.

7. The insufflation apparatus according to claim 1, further comprising a flow-rate changing section that is disposed on the first conduit and changes a flow rate of the predetermined gas, wherein
the control section sets a flow rate allowed to pass by the flow-rate changing section to 0, performs control for setting the switching unit to a state in which the suction-conduit connecting section communicates with the second conduit, sets the suction source in an operation state, and controls the switching unit to operate in a fourth operation mode for sucking the predetermined gas in the subject to the suction source side through the second conduit.

8. The insufflation apparatus according to claim 1, further comprising:
a filter mounting section that is provided in the suction-conduit connecting section and to which a filter that filters the predetermined gas is detachably mountable;
a filter detecting section that detects presence or absence of the mounting of the filter; and
a notifying section that notifies a detection result by the filter detecting section.

9. The insufflation apparatus according to claim 1, further comprising:
a filter that filters the predetermined gas flowing through the suction conduit from the subject;
a characteristic-deterioration detecting section that detects deterioration in a passage characteristic for allowing the predetermined gas to pass by the filter; and
a notifying section that performs notification when the passage characteristic detected by the characteristic-deterioration detecting section is deteriorated to a predetermined value or less.

10. The insufflation apparatus according to claim 1, comprising: a gas feeding tube and a suction tube in which the gas feeding conduit, one end of which is connected to the gas-feeding-conduit connecting section, and the suction conduit, one end of which is connected to the suction-conduit connecting section, are respectively formed by flexible tubes; a gas feeding & suction tube in which at least other end sides of the gas feeding tube and the suction tube are integrated; and a tube connector provided with a cap for gas feeding tube connection and a cap for suction tube connection simultaneously detachably connectable respectively to a cap receiver for gas feeding tube connection and a cap receiver for suction tube connection formed at other ends of the gas feeding & suction tube and respectively forming the gas-feeding-conduit connecting section and the suction-conduit connecting section.

11. The insufflation apparatus according to claim 1, further comprising:
a flow-rate changing section that is provided on the first conduit and changes a flow rate of the predetermined gas; a flow rate sensor provided between the flow-rate changing section and the gas-feeding-conduit connecting section on the first conduit; a pressure sensor provided between the flow-rate changing section and the gas-feeding-conduit connecting section on the first conduit; and an on-off valve that is provided on the connection conduit and performs opening and closing of the predetermined gas flowing in the connection conduit or a check valve that is provided on the connection conduit and feeds the predetermined gas only in one direction from the pump side to the connection conduit side;
a housing in which the gas-feeding-source connecting section, the suction-source connecting section, the gas-feeding-conduit connecting section, the suction-conduit connecting section, the first conduit, the flow-rate changing section, the flow rate sensor, the pressure sensor, the second conduit, the connection conduit, the pump, the on-off valve or the check valve, the switching unit, and the control section are provided;
the suction source disposed on an outside of the housing;
a second conduit for circulation, one end of which is connected to an exhaust port for exhausting the predetermined gas after the suction by the suction source and another end of which is connected to the first conduit in which the flow-rate changing section and the flow rate sensor merge; and
a switching valve that is provided on the second conduit for circulation and selectively causes the second conduit for circulation functioning as the exhaust port side to communicate with an exhaust conduit for exhaust to an atmosphere and the first conduit side, wherein
in a case of the first operation mode, the control section performs control to circulate the predetermined gas sucked by the suction source to the subject side via the second conduit for circulation and the first conduit.

12. The insufflation apparatus according to claim 1, further comprising:
a housing in which the gas-feeding-source connecting section, the gas-feeding-conduit connecting section, the first conduit, the suction-conduit connecting section, the pump, the conduit for circulation, the connection conduit, the suction-source connecting section, the second conduit, the switching unit, and the control section are provided;
the suction source disposed on an outside of the housing;
a second conduit for circulation, one end of which is connected to an exhaust port side for exhausting the predetermined gas after the suction by the suction source and another end of which is connected to the first conduit;
an exhaust conduit provided to branch on the second conduit for circulation and for exhausting the predetermined gas passing in the second conduit for circulation to an atmosphere; and
a switching valve that selectively causes the predetermined gas passing in the second conduit for circulation to communicate with one of the first conduit side and the exhaust conduit side, wherein
in a case of the first operation mode, the control section performs control to circulate the predetermined gas sucked by the suction source into the subject via the second conduit for circulation and the first conduit.

13. The insufflation apparatus according to claim 8, further comprising:
a gas feeding source that is connected to the gas-feeding-source connecting section and feeds the predetermined gas;
the gas feeding conduit that is connected to the gas-feeding-conduit connecting section and feeds the predetermined gas to the subject;
a suction conduit that is connected to the suction-conduit connecting section and sucks the predetermined gas from the subject; and
a flow-rate changing section that is disposed on the first conduit and changes a flow rate of the predetermined gas.

14. The insufflation apparatus according to claim 4, further comprising:
a gas feeding source that is connected to the gas-feeding-source connecting section and feeds the predetermined gas;
the gas feeding conduit that is connected to the gas-feeding-conduit connecting section and feeds the predetermined gas to the subject; and
a suction conduit that is connected to the suction-conduit connecting section and sucks the predetermined gas from the subject.

15. The insufflation apparatus according to claim 5, further comprising:
a gas feeding source that is connected to the gas-feeding-source connecting section and feeds the predetermined gas;
the gas feeding conduit that is connected to the gas-feeding-conduit connecting section and feeds the predetermined gas to the subject; and
a suction conduit that is connected to the suction-conduit connecting section and sucks the predetermined gas from the subject.
